(19) Europäisches Patentamt
European Patent Office
Office européen des brevets

(11) EP 2 985 047 B1

(12) **EUROPEAN PATENT SPECIFICATION**

(45) Date of publication and mention
of the grant of the patent:
**02.09.2020   Bulletin 2020/36**

(51) Int Cl.:
*A61M 5/145* ^(2006.01)   *A61B 6/00* ^(2006.01)
*A61B 6/03* ^(2006.01)   *A61M 5/00* ^(2006.01)
*A61M 5/168* ^(2006.01)   *A61M 5/48* ^(2006.01)

(21) Application number: **14782806.5**

(22) Date of filing: **10.04.2014**

(86) International application number:
**PCT/JP2014/060404**

(87) International publication number:
**WO 2014/168205 (16.10.2014 Gazette 2014/42)**

(54) **CHEMICAL INJECTION DEVICE AND CHEMICAL INJECTION SYSTEM**

CHEMIKALIENEINSPRITZUNGSVORRICHTUNG UND CHEMIKALIENEINSPRITZSYSTEM

DISPOSITIF ET SYSTÈME D'INJECTION DE PRODUIT CHIMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.04.2013   JP 2013083410**

(43) Date of publication of application:
**17.02.2016   Bulletin 2016/07**

(73) Proprietor: **Nemoto Kyorindo Co., Ltd.**
**Tokyo 113-0033 (JP)**

(72) Inventors:
• **NEMOTO, Shigeru**
**Tokyo 113-0033 (JP)**
• **WATANABE, Masakazu**
**Tokyo 113-0033 (JP)**

(74) Representative: **Plasseraud IP**
**66, rue de la Chaussée d'Antin**
**75440 Paris Cedex 09 (FR)**

(56) References cited:
EP-A1- 2 000 161      WO-A1-2007/116864
WO-A1-2011/044706      JP-A- 2006 230 701
JP-A- 2009 056 176      US-A1- 2002 016 569
US-A1- 2003 028 145      US-A1- 2006 184 154
US-A1- 2007 191 770      US-A1- 2010 204 574
US-A1- 2013 041 257      US-B1- 6 673 033

## Description

TECHNICAL FIELD

The present invention relates to a chemical liquid injector and a chemical liquid injection system which are used to inject a chemical liquid, and more specifically, to a chemical liquid injector having a function of monitoring the injection pressure of a chemical liquid during injection thereof and displaying the pressure on a display.

BACKGROUND ART

Currently employed medical imaging diagnostic apparatuses include CT (Computed Tomography) scanners, MRI (Magnetic Resonance Imaging) apparatuses, PET (Positron Emission Tomography) apparatuses, angiography apparatuses, MRA (MR Angiography) apparatuses, ultrasonic imaging diagnostic apparatuses and the like. In using the abovementioned apparatuses, a chemical liquid such as a contrast medium or physiological saline is often injected into a patient in order to provide a better visualization effect.

The chemical liquid is generally filled in a syringe having a cylinder and a piston. The chemical liquid filled in the syringe is typically injected by using a chemical liquid injector. The chemical liquid injector includes a syringe holding mechanism and a piston driving mechanism. While the syringe is held by the syringe holding mechanism, the piston driving mechanism moves the piston forward to inject the chemical liquid filled in the syringe into a patient. The piston driving mechanism typically employs a motor for the driving source.

An injection pressure is one of the criteria to determine whether the chemical liquid is normally injected into the patient or not. When the chemical liquid is normally injected, the injection pressure of the chemical liquid falls within a predetermined range set according to the injection path or the viscosity of the chemical liquid. For example when the chemical liquid leaks somewhere on the chemical liquid injection path, however, the injection pressure of the chemical liquid falls below the predetermined range. In contrast, the injection pressure rises above the predetermined range when the injection path is clogged.

Patent Document 1 has described the real-time detection of the injection pressure of such a chemical liquid, and when the detected injection pressure is out of a predetermined range, an alarm is issued or the operation of a piston driving mechanism is stopped. Patent Document 1 has described, as a means for detecting the injection pressure, a load cell provided for the piston driving mechanism or a means for detection by using a value of current flowing through a motor (hereinafter referred to also as a motor current) which changes depending on a load applied to the piston driving mechanism, and both of them can be used in combination or only one of them can be used. Patent Document 1 has also described the representation of the detected injection pressure in graphical form on a display.

Document US2010204574 discloses an injector according to the preamble of claim 1.

PRIOR ART REFERENCE

PATENT DOCUMENT

Patent Document 1: Patent Application Laid-Open No. 2011-206399

DISCLOSURE OF THE INVENTION

PROBLEMS TO BE SOLVED BY THE INVENTION

Since the detection of the injection pressure based on the result of measurement of the motor current requires no detector such as a load cell for detecting the pressure, such detection can have the advantage of requiring a fewer number of parts as compared with the use of the load cell. However, the following problems arise when the injection pressure is detected by using the result of measurement of the motor current and the operation of the piston driving mechanism is stopped if the detected injection pressure rises above a preset limit pressure.

A first problem is the possibility that the operation may be stopped due to erroneous detection in the initial stage of injection. At the start of the operation of the piston driving mechanism, inrush current flows through the motor. The inrush current is larger as the load on the motor is higher such as when the chemical liquid is injected at a relatively high rate. Depending on the setting of the injection pressure of the chemical liquid, when the inrush current exceeds the motor current corresponding to the upper limit of the injection pressure, the chemical liquid injector determines that the upper limit of the injection pressure is exceeded and stops the operation of the piston driving mechanism.

A second problem is the fact that, once the operation of the piston driving mechanism, or the operation of the motor, is stopped since the injection pressure detected by using the result of measurement of the motor current reaches the predetermined upper limit, no current flows through the motor and thus the injection pressure cannot be measured during the stop of the motor. In the real-time representation of the injection pressure changing over time on the display, the stop of the motor causes zero to be indicated on the graph even when the actual injection pressure is gradually reduced. As a result, a user erroneously assumes that the motor is stopped due to an extremely low injection pressure, rather than due to the injection pressure reaching the predetermined limit pressure.

It is an object of the present invention to provide a chemical liquid injector and a chemical liquid injection system which can eliminate problems such as erroneous detection at start-up and incorrect graph display found in detecting the injection pressure of a chemical liquid by using the result of measurement of a motor current flowing through a motor for driving a piston driving mechanism.

MEANS FOR SOLVING THE PROBLEMS

The invention is defined according to claim 1. The present invention provides a chemical liquid injector injecting a chemical liquid filled in a syringe having a cylinder and a piston, including:

a piston driving mechanism driven by a motor for moving the piston of the syringe into and out of the cylinder;
a motor current measuring circuit configured to measure motor current, the motor current being a current flowing through the motor;
a display device; and
an injector control circuit configured to control operation of the motor such that the chemical liquid in the syringe is injected in accordance with an injection protocol for the chemical liquid, to calculate an injection pressure of the chemical liquid by using the result of the motor current measurement by the motor current measuring circuit, and to display the calculated injection pressure on the display device,
wherein the injector control circuit is configured to temporarily stop the operation of the motor when the calculated injection pressure reaches a preset limit pressure during the injection of the chemical liquid, and to display the preset limit pressure as the injection pressure on the display device instead of the calculated injection pressure during the stop of the operation of the motor due to the injection pressure reaching the preset limit pressure.

EFFECTS OF THE INVENTION

According to the present invention, in the chemical liquid injector in which the result of measurement of the motor current flowing through the motor for driving the piston driving mechanism is used to calculate the injection pressure of the chemical liquid and the calculated injection pressure is displayed on the display device, the injection pressure close to the actual injection pressure can be displayed even when the operation of the motor is stopped since the injection pressure reaches the limit pressure.

BRIEF DESCRIPTION OF THE DRAWINGS

[Fig. 1] A perspective view of an imaging diagnostic apparatus according to an embodiment of the present invention.

[Fig. 2] A front view of a console shown in Fig. 1.

[Fig. 3] A perspective view of an injection head shown in Fig. 1 on which a syringe is mounted.

[Fig. 4] A diagram for showing how the syringe is mounted on the injection head with an adapter interposed.

[Fig. 5] A block diagram of a chemical liquid injector shown in Fig. 1.

[Fig. 6] A section view showing the positional relationship between an RFID tag and an antenna of an RFID module while the syringe is normally held on a cylinder holding mechanism.

[Fig. 7] A graph showing the relationship between time and injection rate from the start of injection operation according to an embodiment of the present invention.

[Fig. 8] A graph showing the relationship between when the operation of a motor is stopped once an injection pressure reaches a limit pressure.

[Fig. 9] A graph showing an example of changes over time in actual injection pressure (Pr), calculated injection pressure (pc), and displayed injection pressure (Pd) according to an embodiment of the present invention.

[Fig. 10A] A plan view of an example of a switch unit usable in the present invention.

[Fig. 10B] A bottom view of the switch unit shown in Fig. 10A.

[Fig. 10C] A front view of the switch unit shown in Fig. 10A.

[Fig. 10D] A back view of the switch unit shown in Fig. 10A.

[Fig. 10E] A right side view of the switch unit shown in Fig. 10A.

[Fig. 10F] A left side view of the switch unit shown in Fig. 10A.

[Fig. 10G] A perspective view of the switch unit shown in Fig. 10A in use.

[Fig. 11] A perspective view showing a particular structure of an example of the syringe usable in the present invention.

[Fig. 12] A perspective view showing a particular structure of another example of the syringe usable in the present invention.

[Fig. 13] A perspective view showing another example of a stand supporting the injection head to which the injection head is attached.

[Fig. 14] A perspective view showing another example of the injection head supported on the stand.

[Fig. 15] A block diagram of a chemical liquid injection system according to another embodiment of the present invention.

[Fig. 16A] An example of a displayed screen when an injection protocol is preset.

[Fig. 16B] An example of a displayed screen when the injection protocol is preset.

[Fig. 16C] An example of a displayed screen when the injection protocol is preset.

[Fig. 17] A diagram showing an example of screen display when the syringe with the RFID tag is mounted and detected.

[Fig. 18] A diagram showing an example of screen display when the syringe with the RFID tag is mounted and detected.

MODES FOR CARRYING OUT THE INVENTION

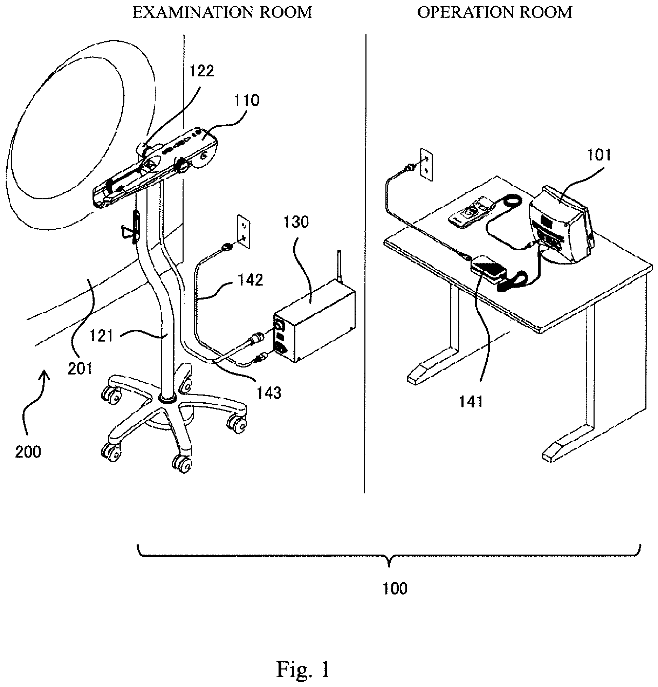

Referring to Fig. 1, an imaging diagnostic system according to an embodiment of the present invention is shown which includes imaging diagnostic apparatus 200 and chemical liquid injector 100. Imaging diagnostic apparatus 200 and chemical liquid injector 100 can be connected to each other so as to allow transmission and reception of data between them. The connection between imaging diagnostic apparatus 200 and chemical liquid injector 100 may be wired or wireless.

Imaging diagnostic apparatus 200 includes scanner 201 configured to perform imaging operation and an imaging control unit (not shown) configured to control the operation of scanner 201 and can obtain tomographic images of a patient injected with a chemical liquid by chemical liquid injector 100. The imaging control unit can include a monitor capable of displaying screens for setting imaging conditions, the obtained tomographic images, and other various types of information relating to imaging.

Chemical liquid injector 100 includes injection head 110 attached to the top of stand 121 through pivot arm 122 and console 101 for controlling the overall operation of chemical liquid injector 100. In the present embodiment, injection head 110 and console 101 are formed as separate units. Stand 121 can be a stand on casters to facilitate the transfer of injection head 110.

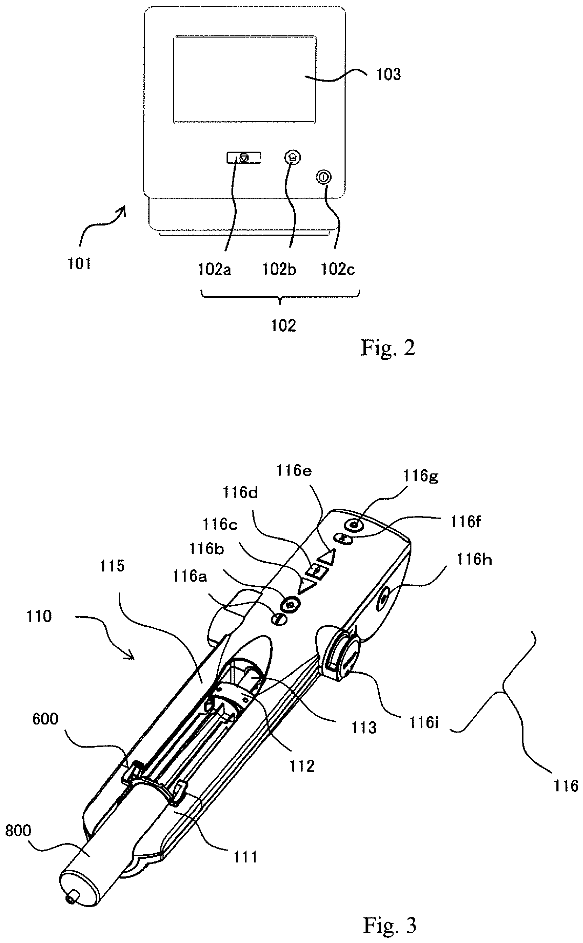

AC adapter 141 is connected to console 101 and is used to supply console 101 with DC power after conversion from AC. As shown in Fig. 2, console 101 has a group of buttons 102 including stop button 102a for forcedly stopping injection, home button 102b for displaying a home screen, and power button 102c for power on/off, and touch panel 103 serving as an input device and as a display device.

As shown in Fig. 3, syringe 800 filled with a contrast medium as a chemical liquid, for example, can be removably mounted on injection head 110. Syringe 800 includes a cylinder containing the chemical liquid and having a conduit formed at a leading end and a piston inserted in the cylinder to be movable into and out of the cylinder. Injection head 110 includes cylinder holding mechanism 111 configured to hold the cylinder and presser 112 operable to push the piston of syringe 800 into the cylinder held in cylinder holding mechanism 111.

Presser 112 is fixed to a leading end of rod 113 which can be moved forward and rearward by an appropriate rotational motion converting mechanism such as a lead screw mechanism and a rack-and-pinion mechanism for converting rotational movement of a motor into linear movement. The mechanism including the motor, rotational motion converting mechanism, rod 113, and presser 112 for moving the piston forward and rearward is referred to as a piston driving mechanism in the present invention. The piston driving mechanism is housed in exterior component 115 made of synthetic resin, except presser 112.

A DC motor can be used as the motor corresponding to the driving source for the piston driving mechanism, and particularly, a brushless DC motor can be used preferably. The brushless motor is quiet and durable due to no brush. Since the brushless motor allows faster rotation, the current required for injecting the chemical liquid at a desired injection pressure can be reduced as compared with a brush motor by increasing an external gear ratio to reduce torque applied to the motor. The brushless motor generally has a sensor such as a Hall sensor for detecting the position of an internal magnet. The output from the sensor can be used to readily provide the rotation amount and the rotation speed of the motor. The rotation amount and the rotation speed of the motor correspond to the position and the moving speed of presser 112, which means that the sensor of the motor can be used to detect the injection amount and the like of the chemical liquid. This eliminates the need of any sensor in injection head 110 dedicated to detection of the injection amount and the like of the chemical liquid, so that the configuration of injection head 110 can be simplified.

Injection head 110 is entirely covered with housing 115 made of synthetic resin except part of the piston driving mechanism (for example, presser 112). Several operation buttons 116 are disposed on an upper face of housing 115 to allow the operation of the piston driving mechanism through the manipulation by a user.

Operation buttons 116 on injection head 110 of the present embodiment include check button 116a operated to enter an injection-ready state, start button 116b operated to start injection, forward button 116c operated to move presser 112 forward by an arbitrary distance (for example, at an injection rate of 1.5 ml/sec), acceleration button 116d operated to increase the moving speed of presser 112 (for example, an addition of 8 ml/sec to the current injection rate, either forward or rearward), rearward button 116e operated to move presser 112 rearward by an arbitrary distance (for example, at an injection rate of 1.5 ml/sec), auto-return button 116f operated to move presser 112 rearward to an initialization position, stop buttons 116g, 116h operated to manually stop or interrupt the operation, and route switch 116i operated to move the presser forward/rearward at a low rate (for example, 0.7 ml/sec).

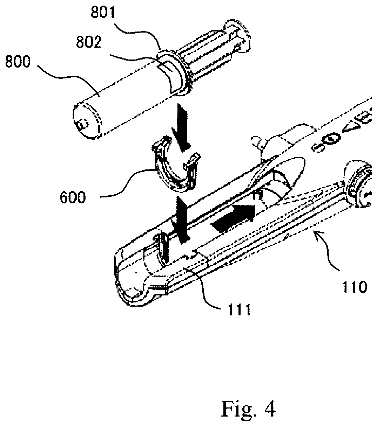

Cylinder holding mechanism 111 is configured to hold syringe 800 mounted thereon with adapter 600 interposed. Syringes 800 of varying sizes are present depending on the capacity of chemical liquid to be contained. Adapter 600 is provided for each of those sizes of syringe 800. As shown in Fig. 4, adapter 600 is configured to hold cylinder flange 801 formed at a trailing end of the cylinder of associated syringe 800 and is removably mounted on cylinder holding mechanism 111 of injection head 110.

The mounting of syringe 800 onto injection head 110 can be performed, for example by mounting adapter 600 on cylinder holding mechanism 111 of injection head 110 and then holding cylinder flange 801 of syringe 800 onto adapter 600. Adapter 600 has a groove for receiving cylinder flange 801, and cylinder flange 801 is inserted into the groove to hold syringe 800 on adapter 600. A lock mechanism may be provided and used such that, after cylinder flange 801 is inserted into the groove of adapter 600, syringe 800 is rotated about its axis by a predetermined angle (for example, 90 degrees) to lock the cylinder. Adapter 600 is used in this manner to allow the mounting of syringes 800 of varying sizes on injection head 110.

Syringe 800 may be a syringe of a pre-filled type which is provided by a manufacturer with chemical liquid already filled therein or may be a syringe of a field-filled type which can be filled with chemical liquid in medical sites.

Referring again to Fig. 1, chemical liquid injector 100 can also include power-supply box 130 formed as a separate unit from injection head 110. Power-supply box 130 converts AC power input thereto through power cable 142 into DC power and supplies the converted DC power through head cable 143 connecting injection head 110 with power-supply box 130.

In injecting the chemical liquid into a patient and obtaining images, scanner 201, injection head 110, and power-supply box 130 of the components described above are placed in an examination room, and the imaging control unit of imaging diagnostic apparatus 200 and console 101 of chemical liquid injector 100 are placed in an operation room separated from the examination room by a wall. Thus, transmission and reception of signals and data between console 101 and injection head 110 are preferably performed through wireless communication. It goes without saying that wired communication can be performed by forming a path for a cable in the wall separating the examination room from the operation room and routing the cable through the path. In the present embodiment, power-supply box 130 is provided with the wireless communication function such that signals are transmitted and received between console 101 and injection head 110 through power-supply box 130.

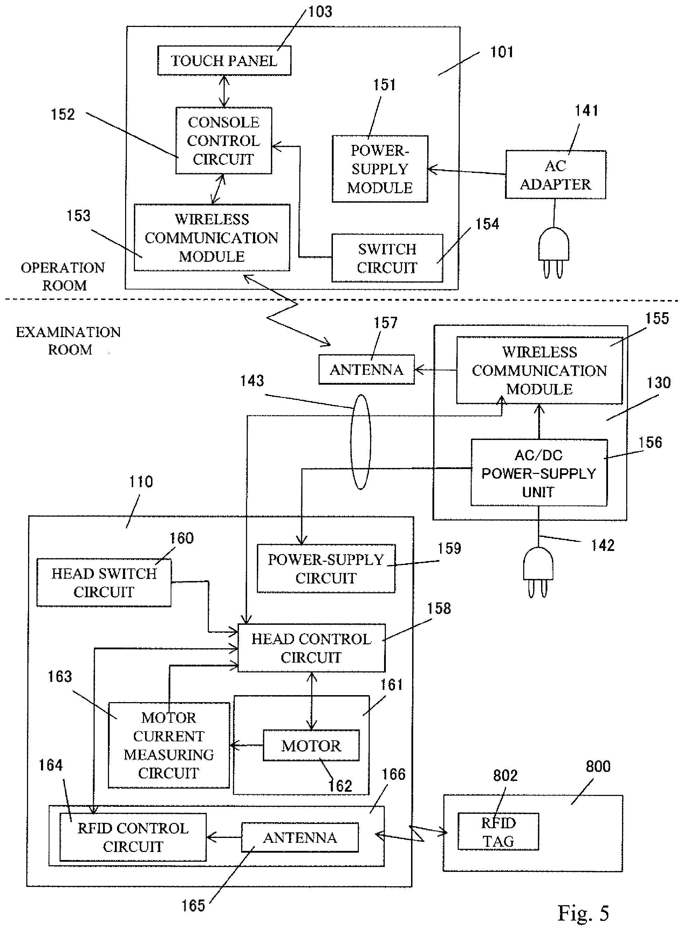

The functions of chemical liquid injector 100 mentioned above will hereinafter be described with reference to a block diagram shown in Fig. 5. Fig. 5 shows only the main functions of chemical liquid injector 100 and the present invention is not limited thereto.

Power-supply box 130 includes AC/DC power-supply unit 156 and wireless communication module 155. AD/CD power-supply unit 156 converts AC power supplied from a commercial power source through power cable 142 into DV power and supplies the DC power to wireless communication module 155 and injection head 110. Wireless communication module 155 is provided with antenna 157 for communication and assists transmission and reception of signals between console 101 and injection head 110. Although the present embodiment is configured such that power-supply box 130 performs wireless communication with console 101 and wired communication with injection head 110 through head cable 143, power-supply box 130 may also perform wireless communication with injection head 110, or wireless communication module 155 may be contained in injection head 110 to allow direct wireless communication between console 101 and injection head 110.

Console 101 includes power-supply module 151, console control circuit 152, wireless communication module 153, switch circuit 154, and touch panel 103 described above.

Power-supply module 151 supplies DC power supplied through AC adapter 141 to console control circuit 152, wireless communication module 153, switch circuit 154, and touch panel 103. Switch circuit 154 includes the group of buttons 102 shown in Fig. 2 and a driving circuit therefor, and transmits signals associated with the operation of the group of buttons 102 to console control circuit 152. Wireless communication module 153 includes an antenna (not shown), and transmits signals transmitted from injection head 110 to console control circuit 152 and transmits signals transmitted from console control circuit 152 to injection head 110 through wireless communication module 155 of power-supply box 130.

In response to input of signals from switch circuit 154 (the operation of the group of buttons 102), input of signals from wireless communication module 153, and input of signals from touch panel 103, console control circuit 152 controls screens, data and the like displayed on touch panel 103, creates an injection protocol for chemical liquid based on data input from touch panel 103 and data transmitted from injection head 110, and transmits data relating to the created injection protocol to injection head 110 through wireless communication module 153 and power-supply box 130.

Console control circuit 152 can be formed as a so-called one-chip microcomputer and can include hardware such as a CPU, ROM, RAM, and interface. The ROM has a computer program stored therein, and the CPU controls each of the components of console 101 by performing various types of processing operations in accordance with the computer program.

Touch panel 103 is provided by modularizing a display serving as a display device, a touch screen serving as an input device, and a control circuit therefor, causes predetermined screens and data based on signals transmitted from console control circuit 152 to be shown on the display, and transmits input information based on signals detected by the touch screen to console control circuit 152. The display can be provided by using any display including a liquid crystal display and an organic electroluminescent display.

Injection head 110 includes head control circuit 158, power-supply circuit 159, head switch circuit 160, piston driving mechanism 161 including motor 162, motor current measuring circuit 163, and RFID module 166.

Power-supply circuit 159 supplies DC power supplied from power-supply box 130 through head cable 143 to head control circuit 158, head switch circuit 160, motor 162, motor current measuring circuit 163, and RFID module 166. Head switch circuit 160 includes operation buttons 116 shown in Fig. 3 and a driving circuit therefor, and transmits signals associated with the operation of operation buttons 116 to head control circuit 158.

RFID module 166 includes RFID control circuit 164 and antenna 165. In the present embodiment, syringe 800 has RFID tag 802 serving as a data carrier attached to the outer circumferential surface of the cylinder (see Fig. 4). RFID module 166 reads information recorded on RFID tag 802 provided as an IC tag and transmits the read information to head control circuit 158. RFID module 166 may also have the function of writing information transmitted from head control circuit 158 to RFID tag 802. RFID control circuit 164 controls the transmission/reception operation of RFID module 166. In other words, RFID module 166 serves as a reader for reading the information from RFID tag 802 or as a reader/writer for additionally writing the information to RFID tag 802.

RFID module 166 can output 200 mW, for example. This allows detection of information in a wider range and transmission/reception of information through the chemical liquid filled in syringe 800.

The information recorded on RFID tag 802 includes, for example, the manufacturer, type, product number, ingredients (particularly, the iodine concentration when the chemical liquid is a contrast medium), fill amount, lot number, and expiration date of the chemical liquid filled in syringe 800, as well as the manufacturer, unique identification number such as product number, allowable pressure value, capacity, piston stroke, dimensions of each component required, and lot number of the syringe. At least some of the information can be transmitted to imaging diagnostic apparatus 200 through a wired or wireless connecting means for transmission and reception of data between imaging diagnostic apparatus 200 and chemical liquid injector 100.

RFID control circuit 164 can be installed at an arbitrary position of injection head 110, but antenna 165 is preferably located at a position facing RFID tag 802 when syringe 800 is normally held in cylinder holding mechanism 111.

Particularly in the present embodiment, as shown in Fig. 4, RFID tag 802 is shaped to have a longitudinal direction and is attached with the longitudinal direction consistent with the circumferential direction of syringe 800. After insertion into the groove of cylinder holding mechanism 111, syringe 800 is rotated to a particular orientation to be normally held. Syringe 800 is designed to have RFID tag 802 facing downward in that held state.

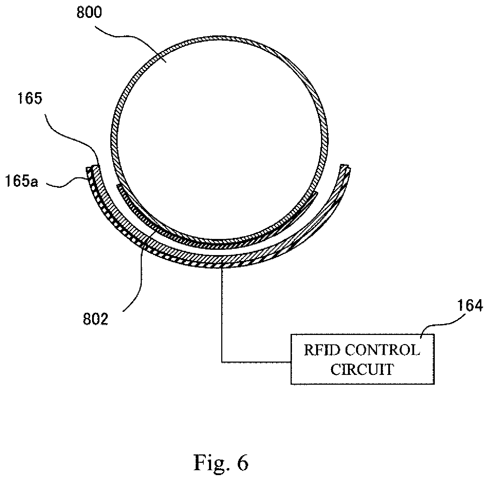

Antenna 165 of RFID module 166 includes a flexible printed circuit (FRC) having a predetermined pattern made of conductor (for example, a pattern having a single loop or a plurality of loops). As shown in Fig. 6, antenna 165 is bent and located at a position facing RFID tag 802 in an arc shape concentric with syringe 800. This intends to cover a wider range in detecting FRID tag 802 attached onto the curved surface.

In the present embodiment, to ensure that RFID tag 802 faces antenna 165 even when the position of attachment of RFID tag 802 varies, antenna 165 has an area larger than that of RFID tag 802. The size of antenna 165 is preferably designed by taking account of the variations in the position of attachment of RFID tag 802 to syringe 800.

As antenna 165 bent in the arc shape has a smaller radius of curvature, and as antenna 165 has a greater length in the circumferential direction, radio waves for communication are more likely to interfere to reduce the communication sensitivity. To reduce the interference of radio waves, antenna 165 preferably has ferrite sheet 165a on the surface of the FPC opposite to the surface facing RFID tag 802.

Referring again to Fig. 5, head control circuit 158 controls, in accordance with input from head switch circuit 160 (the operation of operation buttons 116) and the injection protocol transmitted from console control circuit 152 of console 101, the overall operation of injection head 110 such as the operation of motor 162 serving as the driving source for presser 112 (see Fig. 3) of piston driving mechanism 161 and transmission of the information read from RFID tag 802 by RFID module 166 to console control circuit 152.

Head control circuit 158 can be formed as a so-called one-chip microcomputer and can include hardware such as a CPU, ROM, RAM, and interface. The ROM has a computer program stored thereon, and the CPU controls each of the components of injection head 110 by performing various types of processing operations in accordance with the computer program.

One of the considerations in injecting the chemical liquid filled in syringe 800 into the patient by chemical liquid injector 100 is the withstand pressure values of syringe 800 and the injection path from syringe 800 to the patient (including a tube and an injection needle). When the chemical liquid has a high viscosity, when the chemical liquid is injected at a high rate, and when the injection path has a small diameter, a high pressure is applied to syringe 800. If the injection pressure of the chemical liquid exceeds the withstand pressure values, syringe 800 may be broken or the chemical liquid may leak from the injection path.

Chemical liquid injector 100 of the present embodiment is configured to allow the real-time detection and monitoring of the injection pressure of the chemical liquid such that the injection pressure of the chemical liquid does not exceed the withstand pressure value of syringe 800. The injection pressure is detected by using a motor current, which is the value of current flowing through motor 162. To measure the motor current, injection head 110 also includes motor current measuring circuit 163 for measuring the motor current.

When presser 112 (see Fig. 3) operated with motor 162 as its driving source is moved forward to push the piston of syringe 800 into the cylinder to inject the chemical liquid, the injection pressure is proportional to the magnitude of the load acting on motor 162. In other words, as the injection pressure is higher, the load acting on motor 162 is greater. The magnitude of current supplied to motor 162 for rotating motor 162 is proportional to the magnitude of the load acting on motor 162. Thus, the injection pressure of the chemical liquid is proportional to the motor current, and the injection pressure can be calculated from the motor current.

In the present embodiment, the motor current measured in motor current measuring circuit 163 is transmitted as data to head control circuit 158, and head control circuit 158 also has the function of calculating the injection pressure based on the result of the motor current measurement transmitted from motor current measuring circuit 163.

The calculation of the injection pressure is performed by factoring in a no-load current of motor 162 and a proportionality constant for associating the motor current measurement value with the injection pressure. The no-load current is larger as the number of revolutions of motor 162 is higher. To determine the motor current depending on the load more accurately, the no-load current is preferably subtracted from the actual motor current. Injection pressure P can be determined from the following equation:

$$P = a(C1\text{-}C0)$$

where P represents the injection pressure, C1 represents the motor current value when motor 162 rotates at a number of revolutions, C0 represents the no-load current value at the number of revolutions, and a represents the proportionality constant.

Head control circuit 158 transmits the calculated injection pressure to console control circuit 152, and console control circuit 152 transmits the transmitted data of the injection pressure to touch panel 103 for display on touch panel 103. A combination of the function of console control circuit 152 and the function of head control circuit 158 corresponds to an injector control circuit in the present invention.

Next, the operation of chemical liquid injector 100 is described in more detail. In chemical liquid injector 100 of the present embodiment, operations in a preparatory stage for chemical liquid injection such as holding of syringe 800 and removal of air from syringe 800 and the injection path are performed as conventional, so that the following description is focused on the operation performed after the preparatory stage is completed.

At the completion of the preparatory stage, presser 112 has abutted on a trailing end of the piston of syringe 800, console control circuit 152 has created the injection protocol by using the injection rate, injection amount, injection time period and the like as parameters based on the information acquired from RFID tag 802, the data input through touch panel 103 and the like, the injection path from syringe 800 to the patient has been established, and a limit pressure has been set as a reference used in limiting the injection pressure to prevent a pressure exceeding the allowable pressure from being applied to the equipment used in injection (such as syringe 800, the tube, and the injection needle). The created injection protocol can be displayed on touch panel 103 in the form of graphics or numerical data. The injection protocol has been approved by an operator.

Then, when the operator manipulates the start button of the operation buttons on injection head 110, a signal is transmitted in response thereto from head switch circuit 160 to head control circuit 158. Head control circuit 158 issues an injection protocol data transmission request command to console control circuit 152. In accordance with the request command, console control circuit 152 transmits injection protocol data to head control circuit 158. Upon reception of the injection protocol data, head control circuit 158 drives motor 162 of piston driving mechanism 161 based on the data, thereby allowing the chemical liquid filled in syringe 800 to be injected into the patient.

The injection protocol specifies which chemical liquid should be injected in which amount and at which rate. The injection rate may be constant or may change over time. For injecting a plurality of types of chemical liquids such as a contrast medium and physiological saline, the injection protocol also includes the information representing in which order those types of chemical liquids should be injected. A known injection protocol can be used as the injection protocol. A known procedure may be used as a procedure of creating the injection protocol.

When the contrast medium, for example, is injected as the chemical liquid, the visualization effect produced by the contrast medium varies among patients. To know the range of the variations in visualization effect among individuals, chemical liquid injection involves test injection performed prior to main injection for obtaining tomographic images in imaging diagnostic apparatus 200. The test injection includes injecting the chemical liquid in an injection amount smaller than the amount in the main injection for imaging. Based on the results of the test injection, the determination of timing in the imaging or the like is performed.

In such a case, the chemical liquid injection operation after the test injection can be performed by console control circuit 152 receiving a command transmitted from imaging diagnostic apparatus 200. For example, imaging diagnostic apparatus 200 can obtain tomographic images displayed on a monitor of imaging diagnostic apparatus 200 with a CCD camera (not shown), monitor the brightness (whiteness) in ROIs of the tomographic images, and transmit the command for starting the injection operation to console control circuit 152 when the brightness is equal to or higher than a predetermined threshold value, or measure the strength of a signal from a cable connected to the monitor, and transmit the command similarly when the measurement result is equal to or higher than a predetermined threshold value. When the test injection is performed prior to the injection for obtaining tomographic images (main injection), the CT value and Time Density Curve (TDC) during the test injection can be monitored, the injection protocol can be determined in accordance with the monitoring results, and the optimal imaging start can be issued from chemical liquid injector 100 to imaging diagnostic apparatus 200.

During the injection operation, the motor current is repeatedly measured by motor current measuring circuit 163 at regular time intervals and is transmitted as data to head control circuit 158 each time the measurement is performed. Head control circuit 158 uses the transmitted data of the motor current to calculate the injection pressure as a measurement value. The calculated injection pressure is transmitted as data to console control circuit 152.

In console 101, the limit pressure has been set on the basis of data input through touch panel 103 and data acquired from RFID tag 802 by RFID module 166 and transmitted through head control circuit 158. The set limit pressure is transmitted to head control circuit 158 prior to the injection operation. The injection pressure transmitted from head control circuit 158 is displayed on touch panel 103 in the form of numerical value or injection graph (for example, a graph showing the injection pressure over time with the horizontal axis representing time and the vertical axis representing the injection pressure).

While the chemical liquid injection operation is performed, the calculated injection pressure is compared with the limit pressure transmitted from console control circuit 152 in head control circuit 158. If the injection pressure does not exceed the limit pressure during the injection operation (and when no other error occurs), head control circuit 158 performs the chemical liquid injection operation by driving motor 162 in accordance with the injection protocol from console control circuit 152.

If the comparison between the injection pressure and the limit pressure shows the injection pressure reaches the limit pressure, head control circuit 158 temporarily stops the operation of motor 162, and resumes the operation of motor 162 after the elapse of a predetermined time period. The stop of the operation of motor 162 gradually reduces the injection pressure. The time period during which motor 162 is temporarily stopped can be set to such a time period that the subsequent resumption of the operation of motor 162 does not cause the pressure to reach the limit pressure, for example 0.1 to 1 second, and preferably, 0.2 to 0.5 seconds. The driving conditions for motor 162 at the resumption of the operation of motor 162 are set to the driving conditions after the elapse of the time period during which motor 162 is stopped. When the temporary stop and the resumption of the operation of motor 162 are repeated as described above, and the injection pressure is not reduced, for example after five seconds of such repetitions, head control circuit 158 may determine that the injection path from syringe 800 to the patient is clogged, and stop the chemical liquid injection operation, that is, the operation of motor 162.

The temporary stop of the operation of motor 162 when the injection pressure reaches the limit pressure controls the chemical liquid injection operation such that the injection pressure does not exceed the limit pressure. Although the set value of the limit pressure can be changed arbitrarily by the operator as required, an ultimate pressure is often set for chemical liquid injector 100 aside from the limit pressure. The ultimate pressure is set fixedly for the chemical liquid injector as a safety means used when the limit pressure is exceeded due to some abnormality, by way of example, and cannot be changed by the operator. The limit pressure is set to be a value smaller than the ultimate pressure.

The above is the basic flow of the chemical liquid injection operation. In the present embodiment, the following operation is further performed.

Firstly, at start-up of piston driving mechanism 161, that is, at start-up of motor 162, for example at the start of the chemical liquid injection, head control circuit 158 does not drive motor 162 to reach a target speed immediately after the start-up but increases the number of revolutions of motor 162 over time to reach the target speed in a predetermined time period after the start-up.

An inrush current several times larger than the rating flows through motor 162 at the start-up, and motor 162 operates with a normal current after it begins rotating. Motor current measuring circuit 163 measures not only the motor current value during the injection operation but also the inrush current, and outputs it as current value data to head control circuit 158. Head control circuit 158 measures the injection pressure based on the inrush current input from motor current measuring circuit 163.

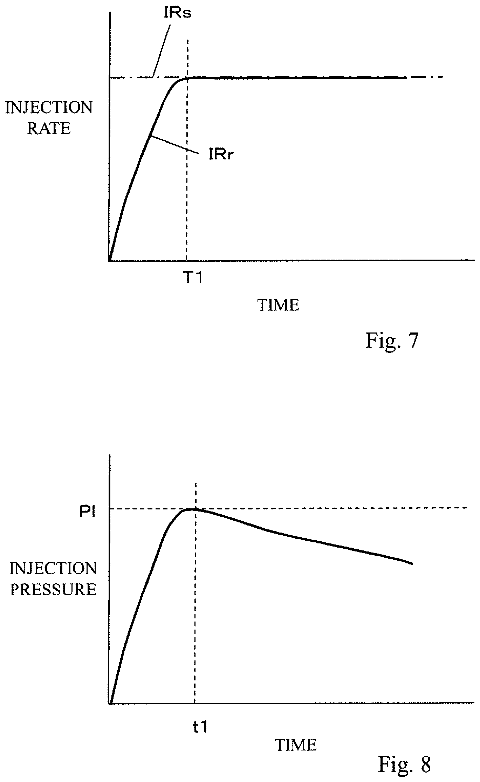

Depending on the magnitude of the inrush current, the calculated injection pressure may exceed the limit pressure, and consequently, motor 162 may be stopped before chemical liquid injection and thus no chemical liquid may be injected. To address this, in the present embodiment, as shown in Fig. 7, the number of revolutions of motor 162 is increased over time such that an actual injection rate (IRr) reaches a target injection rate (IRs) at a target reach time (T1) which is a point in time after the elapse of a predetermined time period since the start of injection. This can prevent the stop of the injection operation due to the inrush current.

The target reach time (T1) at which the actual injection rate (IRr) reaches the target injection rate (IRs) may be a time at which the inrush current through motor 162 does not affect the calculation of the injection pressure, and can be set arbitrarily in accordance with the characteristics of motor 162 and the injection conditions such as the injection amount and injection time period of the chemical liquid. Specifically, the target reach time (T1) may be at 1 to 2 seconds. The control performed by head control circuit 158 at the start-up of the motor is performed not only at the start of injection but also in all cases where motor 162 is started from a standstill. For example, when the injection protocol includes a time interval during which chemical liquid injection is temporarily stopped, the control is applicable in the resumption of chemical liquid injection after the interval. When the injection protocol includes the injection rate changing over time, the target injection pressure (IRs) is set to the injection pressure at the target reach time (T1).

Secondly, console control circuit 152 controls the display of the chemical liquid injection rate on touch panel 103 during the injection operation in response to ON/OFF of motor 162.

When the result of the motor current measurement is used to calculate the chemical liquid injection pressure, and the operation of motor 162 is temporarily stopped if the calculated injection pressure reaches the preset limit pressure, no motor current flows during the stop of the operation of motor 162 and thus the calculation of the injection pressure results in zero. The following phenomenon occurs when the injection pressure during the injection operation is displayed on touch panel 103 in real time as the graph or numerical values.

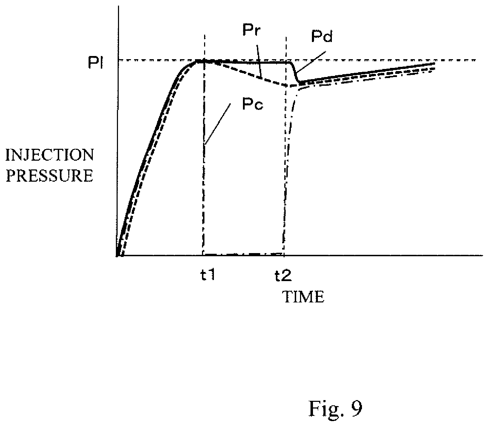

For example, as shown in Fig. 8, when the injection pressure reaches a limit pressure Pl at a time t1 after the start of injection, and the operation of motor 162 is stopped, then the injection pressure is gradually reduced. As shown in Fig. 9, however, an injection pressure Pc calculated by using the result of the motor current measurement becomes zero in response to the stop of the operation of motor 162, and significantly deviates from the actual injection pressure.

In the present embodiment, as shown in Fig. 9, console control circuit 152 controls the display on touch panel 103 such that the limit pressure (Pl) is displayed as the injection pressure instead of the calculated injection pressure (Pc) during the period (the period from the time t1 to a time t2) in which the operation of motor 162 is temporarily stopped after the calculated injection pressure (Pc) reaches the limit pressure (Pl), and the injection pressure (Pc) calculated by using the result of the motor current measurement is displayed after the restart of motor 162. In Fig. 9, Pr represents the actual injection pressure and Pd represents the injection pressure displayed on touch panel 103.

Since the inrush current flows at the restart of motor 162, head control circuit 158 controls motor 162 by taking the inrush current into account as described above in restarting motor 162. Console control circuit 152 switches the display of the injection pressure on touch panel 103 from the limit pressure (Pl) to the calculated injection pressure (Pc) at the time when the injection rate reaches a target injection pressure. With regard to how the injection graph looks, the boundaries before and after the switching may be complemented by curved lines to avoid the injection graph looking unnatural before and after the switching of the display. Although Fig. 9 shows the example in which touch panel 103 displays the injection graph having the horizontal axis showing time and the vertical axis showing the injection pressure for representing the injection pressure over time, the above description is preferably applied to the case where the injection pressure is displayed as numerical values, when applicable.

Since the display on touch panel 103 is controlled in this manner, the injection pressure closer to the actual pressure can be displayed even during the temporary stop of motor 162. As a result, the operator can readily determine that the stop of the operation of motor 162 during the injection operation is performed in order to prevent the injection pressure from exceeding the limit pressure.

Although the present invention has been described with the representative embodiment, the present invention is not limited to the embodiment described above and can be changed arbitrarily without departing from the spirit or scope of the technical idea of the present invention.

For example, the above embodiment includes measuring the motor current at regular time intervals and calculating the injection pressure at each measurement. Alternatively, measurement values may be accumulated over a plurality of (for example, 5 to 10) preceding measurements and then the injection pressure may be calculated from the average of the accumulated values over the plurality of measurements. This can calculate the injection pressure closer to the actual injection pressure by reducing the influence of errors or variations in the measurement values.

The calculated injection pressure may be used for an alarm issued when empty syringe 800 containing no chemical liquid is erroneously mounted on injection head 110 and the injection operation is performed or when the injection operation is performed without mounting syringe 800. In these cases, an extremely small pressure is measured. For example when the injection rate is set at 3.0 ml/sec or higher, and injection operation for about 15 ml or more is performed with a measured pressure of $49 \times 10^3$ Pa or lower, head control circuit 158 or console control circuit 152 determines that the injection operation is performed with empty syringe 800 or that the injection operation is performed without mounting any syringe, and can perform the associated processing. The associated processing includes, for example, stopping the injection operation, displaying on touch panel 103 (see Fig. 2) the fact that the empty syringe is mounted or no syringe is mounted, and issuing an alarm of sound or voice from a sound generator (not shown) such as a buzzer.

The above embodiment includes console control circuit 152 and head control circuit 158, and the functions relating to the overall control of chemical liquid injector 100 are divided between them. The division of the functions between the control circuits can be changed as appropriate depending on the roles of console 101 and injection head 110. Alternatively, console control circuit 152 and head control circuit 158 can be integrated into a single control circuit which controls the overall operation of chemical liquid injector 100. The integrated control circuit, when used, may be included in console 101 or in injection head 110.

Some or all of the functions of head control circuit 158 including the determination (computation) of the injection protocol and/or some or all of the functions of console control circuit 152 can be incorporated into a unit independent of chemical liquid injector 100, such as imaging diagnostic apparatus 200. Particularly when the function of determining (computing) the injection protocol is incorporated into imaging diagnostic apparatus 200, it is unnecessary that the data common to the setting of imaging conditions and the setting of injection conditions should be input redundantly to both imaging diagnostic apparatus 200 and chemical liquid injector 100. When all of the functions of console control circuit 152 are incorporated into imaging diagnostic apparatus 200, console 101 of chemical liquid injector 100 is not required and the overall system can be simplified. Conversely, the function of setting imaging conditions in imaging diagnostic apparatus 200 can be incorporated into chemical liquid injector 100 to eliminate the need of the console of imaging diagnostic apparatus 200.

The functions of console control circuit 152 and the functions of head control circuit 158 can be realized by using various types of hardware as required, and the main part is realized by a CPU functioning in accordance with a computer program.

The computer program can be implemented as software for performing processing including, at least, the step of measuring the motor current corresponding to the current flowing through motor 162 serving as the driving source for piston driving mechanism 166, the step of calculating the injection pressure based on the result of the motor current measurement, the step of displaying the calculated injection pressure on the display device, the step of temporarily stopping the operation of motor 162 when the calculated injection pressure reaches the preset limit pressure, and the step of displaying the limit pressure as the injection pressure instead of the calculated injection pressure on the display device during the temporary stop of the operation of motor 162.

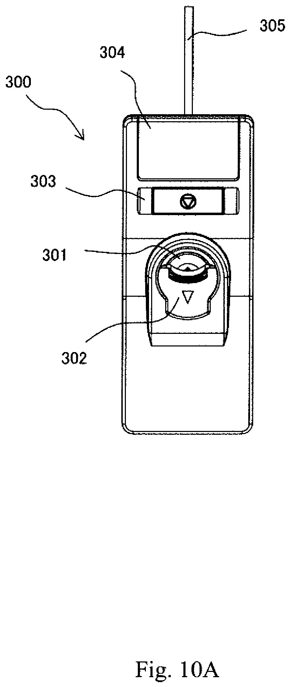

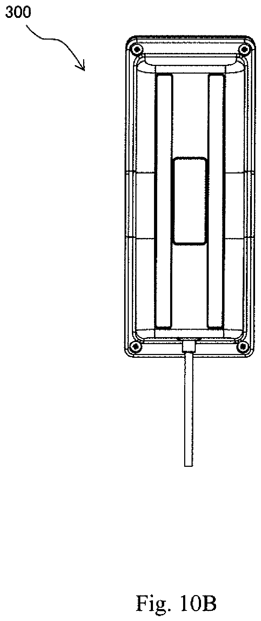

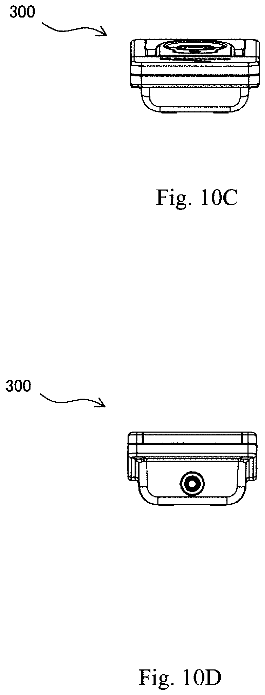

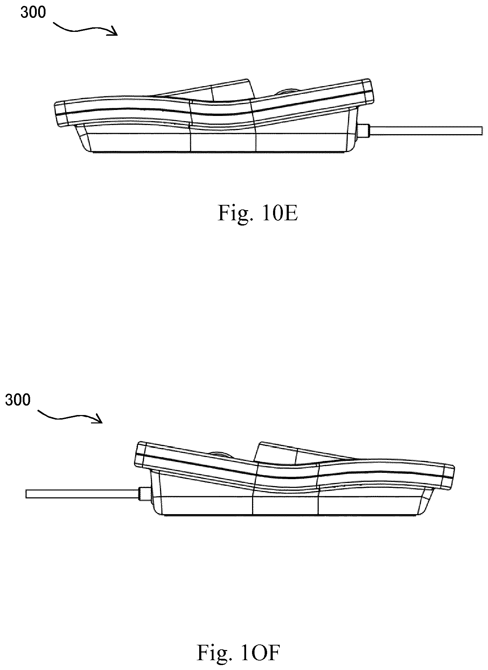

In terms of structure, injection head 110 and power-supply box 130 may be integrally formed, and console 101 may be integrally formed in addition. When console 101 and injection head 110 are integrally formed, the console is also placed in the examination room. For starting and stopping the injection operation, a remote controller is preferably used. The use of the remote controller allows the operator to control the start and stop of the injection operation in the operation room. For example, the remote controller as shown in Fig. 10A to Fig. 10G may be used, as described later in detail.

Although the above embodiment employs the wireless communication technology for part of the transmission and reception of the signals and data, the transmission and reception of all the signals and data may be performed through wired communication.

Chemical liquid injector 100 can further include a switch unit serving as the remote controller to enable the start and stop of the injection operation in the operation room.

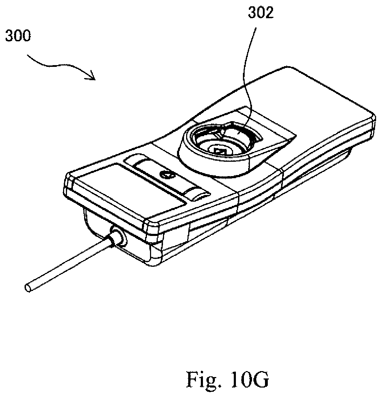

Fig. 10A to Fig. 10G show an example of the switch unit which can be used in the present invention. Switch unit 300 shown in Fig. 10A to Fig. 10G can be connected to console 101 (see Fig. 1) through cable 305. Switch unit 300 includes start button 301 operated to start the injection and stop button 303 operated to stop the injection. Part of start button 301 is covered with slide cover 302 to prevent erroneous operation. As shown in Fig. 10G, slide cover 302 can be opened or closed by manual sliding, and is opened in operating start button 301 and is closed at other times. Switch unit 300 can include display 304. Display 304 can display the injection state, the injection time period and the like of the chemical liquid. The connection of switch unit 300 to console 101 may be made with cable 305 as shown or through wireless connection.

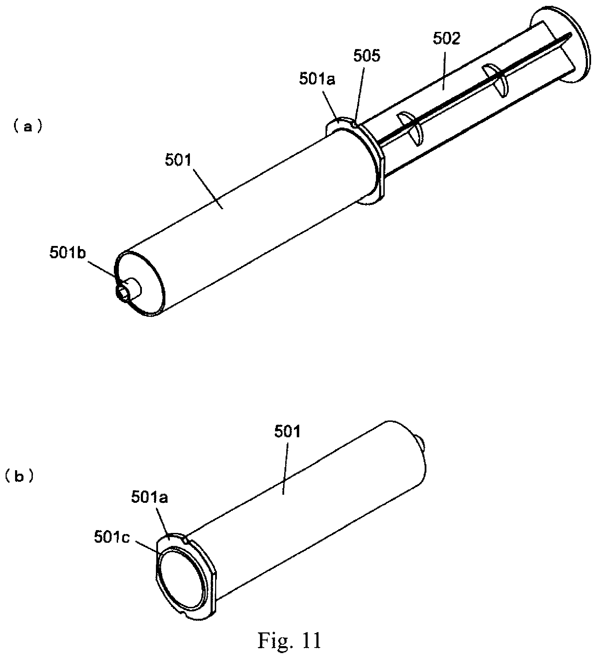

Specifically, a syringe as shown in Fig. 11(a) and Fig. 11(b) may be used, and this syringe is for 100 ml, by way of example. This syringe includes cylinder member 501 and piston member 502. Cylinder flange 501a formed at a trailing end of cylinder member 501 has a profile of I-cut shape, and flange 501a has two notch portions 505 (only one of them is shown) on the outer peripheral portion. Conduit portion 501b at a leading end of cylinder member 501 may be for lure lock connection and have two tubular portions placed concentrically on the inner and outer sides. As shown in Fig. 11(b), cylinder flange 501a may have ring-shaped protruding portion 501c formed on the rearward face.

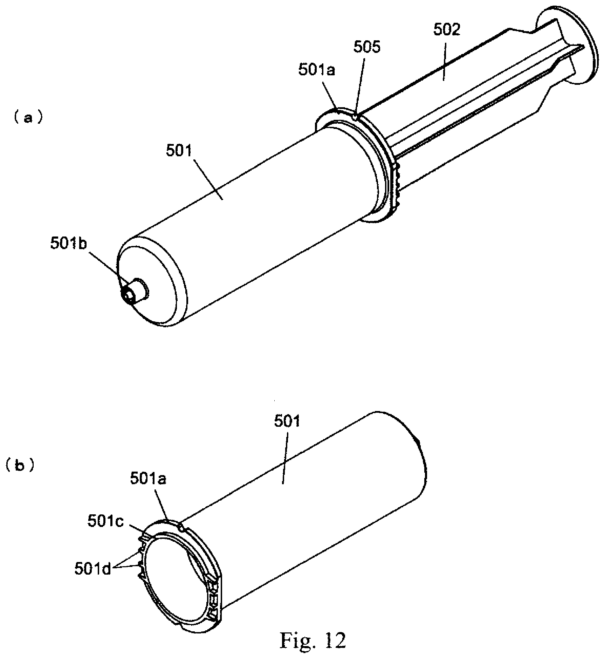

A syringe as shown in Fig. 12(a) and Fig. 12(b) may be used as another example. This syringe is for 200 ml, by way of example. Similarly to the syringe described above, this syringe may include cylinder member 501 and piston member 502, and cylinder flange 501a formed at a trailing end of cylinder member 501 may have a profile of I-cut shape. Cylinder flange 501a has two notch portions 505 (only one of them is shown) on the outer peripheral portion. Conduit portion 501b at a leading end of cylinder member 501 may be for lure lock connection and have two tubular portions placed concentrically on the inner and outer sides. As shown in Fig. 12(b), cylinder flange 501a may have, on the rearward face, ring-shaped protruding portion 501c and a plurality of ribs 501d extending outward from protruding portion 501c.

Although Fig. 12 shows cylinder flange 501a having both notch portions 505 and ribs 501d formed thereon, only one of them may be formed (for example, no notch portions 505 may be formed). Out of ribs 501d arranged vertically in Fig. 12(a) and Fig. 12(b), only two ribs at the top and bottom may be left and the other ribs may be omitted. The syringe may have such a group of ribs formed on only one of the left and right sides of the flange portion.

An adapter on which the syringe having notch portions 505 formed in cylinder flange 501a as shown in Fig. 11 and Fig. 12 is mounted preferably has a groove into which cylinder flange 501a is inserted from above in the orientation as shown and has a protrusion which engages with notch portion 505 after the syringe is rotated about its axis by 90 degrees. This holds the cylinder more securely. Even when a trouble in injection pressure control occurs during the injection operation to apply an excessive pressure to the syringe, cylinder flange 501a is unlikely to be broken since the syringe is tightly held. In addition, unbalanced loading due to inclined mounting of the syringe hardly occurs, and a liquid leak from a gap between the piston and the cylinder can be prevented.

The syringes shown in Fig. 11 and Fig. 12 may include an RFID tag on the outer circumferential surface of the cylinder similarly to syringe 800 described above.

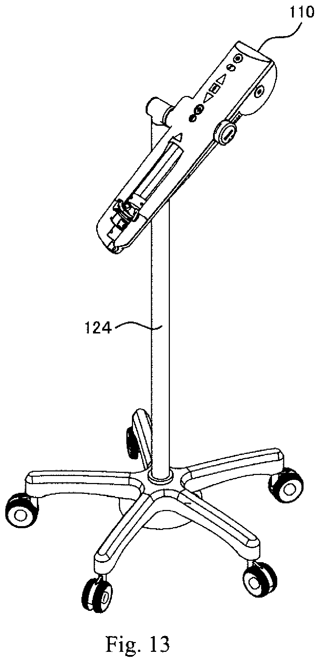

Although Fig. 1 shows that the stand for supporting injection head 110 has a generally S-shaped curve, any stand can be used such as linear stand 124 as shown in Fig. 13. When any stand is used, power-supply box 130 (see Fig. 1) may be fixed to the stand. Since injection head 110 and power-supply box 130 are always used together, the fixing of power-supply box 130 to the stand can facilitate the transfer of them between the rooms. Alternatively, injection head 110 may be supported on an articulated support arm assembly fixed to the ceiling.

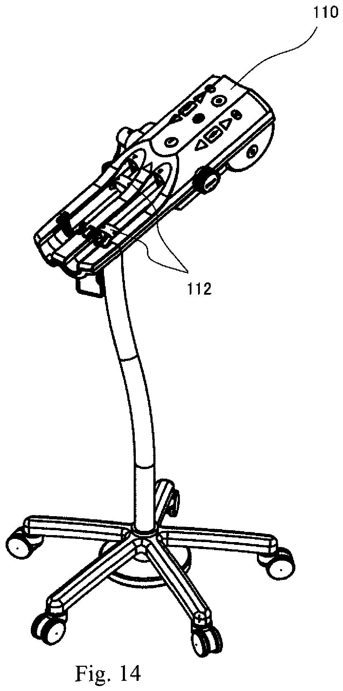

Injection head 110 can also be configured to simultaneously support two or more syringes 800 mounted thereon. By way of example, Fig. 14 shows injection head 110 on which two syringes can be mounted simultaneously. Injection head 110 shown in Fig. 14 includes piston driving mechanisms which can operate independently of each other. Each of the piston driving mechanisms includes presser 112 operative with a motor used as the driving source. Since the two syringes can be mounted simultaneously, various injection procedures can be realized, including mounting a syringe filled with a contrast medium and a syringe filled with physiological saline on injection head 110, injecting the contrast medium and then injecting the physiological saline, and pushing the contrast medium by the physiological saline, or diluting the contrast medium with the physiological saline at a desired concentration for injection.

The speed control for motor 162 at the start-up described above is applicable not only to the chemical liquid injection operation but also to all the operations involving the operation of motor 162. Prior to the chemical liquid injection, "removal of air" is typically performed to remove air bubbles from the syringe and the tube connecting the syringe with the patient. The "removal of air" is performed by operating motor 162 to repeatedly move the piston forward and rearward while the syringe is mounted on the injection head. In this case, the control as described above is performed to increase the number of revolutions of motor 162 over time, so that erroneous operation due to inrush current can be prevented.

In addition, head control circuit 158 may reduce the number of revolutions of motor 162 over time at the stop of motor 162 in contrast to the control of motor 162 at the start-up. This can realize more stable operation and reduce damage to the driving mechanism. In this case, the time period required to stop motor 162 operating at the present speed can be set to 1 to 2 seconds. This operation is applicable to all the cases where rotating motor 162 is stopped, and is particularly effective in stopping presser 112 by simultaneously releasing forward button 116c and acceleration button 116d on injection head 110 after presser 112 is moved forward at the maximum speed by simultaneously pressing these buttons 116c and 116d.

The chemical liquid injector may further include a load cell for detecting the injection pressure. The load cell can be provided for presser 112, for example. When a plurality of pressers 112 are included as shown in Fig. 14, at least one of them may include the load cell. When the load cell is provided, the injection pressure is typically detected by the load cell, and only when the load cell fails, the result of the motor current measurement can be used to measure the injection pressure.

The imaging diagnostic apparatus may be any imaging diagnostic apparatus such as a CT scanner, MRI apparatus, PET apparatus, angiography apparatus, and ultrasonic imaging diagnostic apparatus as long as it may be used with chemical liquid injector 100.

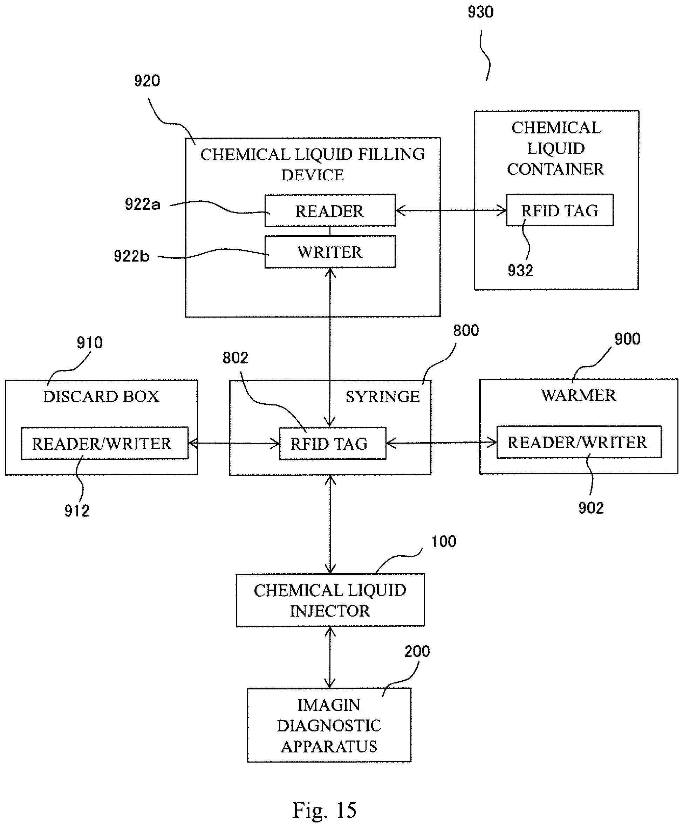

As shown in Fig. 15, the chemical liquid injection system may further include warmer 900 for heating syringe 800 before use to a predetermined temperature and discard box 910 for housing used syringe 800 to be discarded, in addition to chemical liquid injector 100 and imaging diagnostic apparatus 200. Warmer 900 and discard box 910 may be equipment independent of chemical liquid injector 100 and imaging diagnostic apparatus 200, or may be connected to at least one of them via a network to allow data communication. Warmer 900 and discard box 910 may be independent of each other or may be connected to each other via a network to allow data communication. Warmer 900 and discard box 910 can include reader/writer 902 and 912, respectively, for reading information recorded on RFID tag 802 or writing information to RFID tag 802. When syringe 800 is heated by warmer 900, reader/writer 902 writes the information indicating the fact of heating to RFID tag 802 on syringe 800. When used syringe 800 is housed into discard box 910, reader/writer 912 records the information indicating that this syringe 800 is discarded onto RFID tag 802.

The chemical liquid injection system may further include chemical liquid filling device 920. Chemical liquid filling device 920 is an apparatus which can support an empty syringe not filled with any chemical liquid and fill the syringe with chemical liquid. Chemical liquid filling device 920 may be an apparatus independent of chemical liquid injector 100, imaging diagnostic apparatus 200, warmer 900, and discard box 910, or may be connected to at least one of them via a network to allow data communication. The empty syringe with a piston located at the foremost position is connected through a tube or the like to chemical liquid container 930 of any form such as a bag or a bottle containing chemical liquid to achieve fluid communication between them. After the connection of the empty syringe with chemical liquid container 930, chemical liquid filer 920 can move the piston rearward to fill the empty syringe with the chemical liquid. The empty syringe preferably has an RFID tag serving as a data carrier attached thereto. The following description is made assuming that the empty syringe is syringe 800 having RFID tag 802 attached thereto before the filling with the chemical liquid.

Chemical liquid container 930 also has RFID tag 932 serving as a data carrier attached thereto. RFID tag 932 has information relating to the contained chemical liquid recorded thereon as data including the type, amount, manufacturer, product number, viscosity, expiration date, and the iodine concentration when the chemical liquid is a contrast medium. Chemical liquid filling device 920 includes reader 922a capable of reading the data from RFID tag 932 and writer 922b capable of writing the data to RFID tag 802 attached to syringe 800.

In filling the chemical liquid into syringe 800 from chemical liquid container 930 by chemical liquid filling device 920 in the above configuration, the data recorded on RFID tag 932 attached to chemical liquid container 930 is read by reader 922a. Chemical liquid filling device 920 includes a storage such as a memory, and the read data is temporarily stored in the storage. Then, the operator sets a filling amount in chemical liquid filling device 920 and operates chemical liquid filling device 920.

Thus, the set amount of chemical liquid is filled into syringe 800. The filling amount can be set in accordance with a predetermined operation procedure for chemical liquid filling device 920. After the filing of the chemical liquid, writer 922b writes the filling amount and filling date and time of chemical liquid to RFID tag 802 on syringe 800 together with the data temporarily stored in the storage. Through these operations, syringe 800 is filled with the chemical liquid and the data relating to the filled chemical liquid is recorded on RFID tag 802.

RFID tag 802 may have the data relating to the syringe as described above previously recorded thereon. Reader 922a for reading the data from RFID tag 932 on chemical liquid container 930 may be a reader/writer which can additionally write data. In this case, the reader/writer can write, to RFID tag 932, the present amount (remaining amount) calculated by subtracting the filling amount from the amount contained in chemical liquid container 930 before the filling. The calculation of the remaining amount can be performed by a CPU included in chemical liquid filling device 920.

Chemical liquid injector 100 may have a built-in watch circuit for providing the present date and time. In this case, the filling date and time recorded on RFID tag 932 is read in RFID module 166 (see Fig. 5), the read filling date and time is compared with the present date and time provided by the watch circuit in head control circuit 158 (see Fig. 5), and when the comparison indicates that the present date and time is after the elapse of a predetermined time period since the filling date and time, that is, that the expiration date is passed, then head control circuit 158 can perform processing of preventing the injection of the chemical liquid. The processing of preventing the injection of the chemical liquid includes, for example, disabling the operation of piston driving mechanism 161 (see Fig. 5), transmitting a signal indicating that the expiration date of the chemical liquid is passed to console control circuit 152 (see Fig. 5) and causing console control circuit 152 to display the fact that the expiration date of the chemical liquid is passed on touch panel 103 (see Fig. 5), and issuing an alarm of sound or voice from a sound generator (not shown) such as a buzzer. The expiration date of the chemical liquid is preset in chemical liquid injector 100, but the set expiration date can be changed arbitrarily by the operator. The management of the filling date and time of the chemical liquid as described above can ensure the safety of the contrast medium to be injected.

Each of the components constituting the chemical liquid injection system such as chemical liquid injector 100, imaging diagnostic apparatus 200, warmer 900, discard box 910, and chemical liquid filling device 920 may be connected to a medical network. This allows simple storage and track of a history of treatments for a patient, history of uses of the chemical liquid, history of uses of the syringe and the like.

The chemical liquid injection system including at least chemical liquid injector 100 and imaging diagnostic apparatus 200 may be connected to the medical network. This allows the injection rate, injection time period, injection amount of the chemical liquid injected by chemical liquid injector 100, the injection results including the injection graph formed by using the injection pressure calculated on the basis of the result of the motor current measurement, and the imaging conditions in imaging diagnostic apparatus 200 (including the imaging time period and tube voltage when the imaging apparatus is a CT scanner) to be stored as injection data in the imaging diagnostic apparatus, RIS, PACS, and HIS via the medical network. The stored injection data is used for managing the injection history, and particularly, the injection amount can be used for accounting, for example. In addition, the body information of a patient such as the weight, ID, name, examination region, and examination method can be acquired from the RIS, PACS, HIS and the like and displayed on the chemical liquid injector to perform injection appropriate for the acquired information.

The amount of chemical liquid filled by chemical liquid filling device 920 can be set to the amount of chemical liquid to be injected into a patient. This allows use of the filled chemical liquid without waste. The injection amount can be calculated with an equation which factors in the body characteristics of the patient such as the weight, imaging region, and the imaging time period, or the value of the injection amount can be determined directly by a doctor or the like. The factors considered in calculating the injection amount or the value of the injection amount determined by the doctor or the like can be input by the operator or can be obtained from an external database in the RIS, HIS, PACS, external server, or cloud connected over a network or a direct channel. Erroneous input made by the operator can be avoided when the factors used in calculating the injection amount are obtained from the external database.

The calculation of the injection amount with the equation is performed in the console control circuit. The function of the console control circuit may be included in any computer apparatus such as the control circuit included in the chemical liquid injector, the imaging diagnostic apparatus, or the chemical liquid filling device. In other words, the injection amount of chemical liquid can be calculated not by the chemical liquid injector but by another computer apparatus. The function of the console control circuit is included not only in the chemical liquid injector but in another computer apparatus, so that the other computer apparatus can create the injection protocol having the injection rate and the injection time period as parameters in addition to the injection amount of chemical liquid.

In the following, description is made of the specific operation of the chemical liquid injector in conjunction with an example in which injection head 110 capable of supporting two syringes as shown in Fig. 14 is used to inject a contrast medium and physiological saline as the chemical liquid with reference to the block diagram of Fig. 5 and the like.

Touch panel 103 can display a screen for performing normal chemical liquid injection and a screen for performing injection in another injection method or predetermined editing. These modes can be switched through operation of a switch on console 101 or operation of icons displayed on touch panel 103.

First, the mode of performing normal chemical liquid injection is described.

In the mode of performing normal chemical liquid injection, the operator first mounts a syringe filled with contrast medium and a syringe filled with physiological saline onto injection head 110. Each of the syringes has RFID tag 802 attached thereto. When the syringes are mounted, the information recorded on RFID tag 802 is read by RFID control circuit 164. The read information includes, for example, information relating to the amount, iodine concentration, product name of the contrast medium, and the withstand pressure of the syringe. The information relating to the injection protocol is also recorded on RFID tag 802 and may be read from RFID tag 802. Console control circuit 152 may have a storage section in which the information read from RFID tag 802 is stored.

The amount of the contrast medium is, for example, 100 ml, 150 ml, or 200 ml. The iodine concentration is, for example, 240 mgI/ml, 300 mgI/ml, 320 mgI/ml, 350 mgI/ml, or 370 mgI/ml. The withstand pressure of the syringe is, for example, 15 kgf/cm$^2$.

The numerical value associated with the "withstand pressure of syringe" means that injection at a pressure exceeding that numerical value may break the syringe. The numerical value may be automatically read from RFID tag 802 and set as a withstand pressure value of piston driving mechanism 161, or a numerical value different from the withstand pressure value may be set through input by a user. For example when the numerical value read from RFID tag 802 is 15 kg/cm$^2$ and the numerical value input by the user is 20 kg/cm$^2$, then 15 kg/cm$^2$, which is the smaller value, is preferably set with a higher priority.

The read information is sent to console control circuit 152 and is displayed as required on touch panel 103.

(Normal Chemical Liquid Injection Mode)

In the normal chemical liquid injection mode, for example, an image of human body shape horizontally oriented is displayed at the center of the screen of touch panel 103. The human body image can include a plurality of icons representing a head part, a chest part, an abdomen part, and a leg part. An imaging region can be selected by touching the associated icon.

Another group of icons may be displayed under the human body image, but those icons are not displayed in the initial screen and only the human body image is displayed. Although the initial screen may be configured to show both the human body image and the other group of icons, the display of only the human body image simplifies displayed items on the screen to allow more intuitive operation by the user.

When the user touches one of the icons (for example, the icon representing the abdomen part) in the human body image, icons showing imaging regions associated with the abdomen part (including "tumor portion," "liver," "kidney," and "blood vessel" in this case) are displayed. These icons can be displayed horizontally in line under the human body image. Each of the icons has only the picture representing the associated organ and does not have characters such as "tumor portion," "liver," "kidney," or "blood vessel." These characters may or may not be displayed, but when the icon has only the picture without characters, the display is more simplified. Whether or not the characters are displayed may be switched. The selected icon is preferably highlighted. This allows the user to recognize that the body part is being selected.

Next, the imaging region is selected. For example when the icon of the liver is touched, the picture of the liver may be moved into the abdomen part of the human body image as if it is sucked, and then displayed within the abdomen portion.

After the imaging region is selected in this manner, the chemical liquid injection time period associated with the imaging region is read from the storage section of console control circuit 152 and displayed on touch panel 103. For example, the injection time period for the liver is 30 seconds, and the icon showing "0:30 sec" can be displayed in a lower portion on the screen.

A plurality of weight icons may be displayed on touch panel 103 to select the weight of the patient. The plurality of weight icons can be displayed horizontally in line under the human body image. The weight can be classified, for example, into three ranges represented by a light-weight icon for weights of less than 40 kg, an intermediate-weight icon for weights from 40 kg to less than 70 kg, and a heavy-weight icon for weights of more than 70 kg.

The number of the weight ranges may be changeable. The value of the weight of each range may be arbitrarily changeable. A numeric keypad may be displayed in response to a predetermined operation on the weight icon and used to directly input a numerical value.

A weight scale icon showing the picture of a weight scale and a syringe icon showing the picture of a syringe may be displayed between the weight icon and the human body image. When the weight scale icon is touched, the weight can be input specifically (described later in detail). When the syringe icon is touched, switching is made to a "flow rate mode" in which the injection rate can be selected arbitrarily (described later in detail). The syringe icon may be displayed on the initial screen.

Next, the operator touches one of the plurality of weight icons to input the weight of the patient. The following description is made of the case where the intermediate-weight icon for weights from 40 kg to less than 70 kg is touched.

When the weight icon is touched to determine the weight range, the selected single icon is highlighted. As an example of display form, the icon of the injection time period may be moved from the lower portion on the screen to a position near the left of the weight scale icon and displayed at that position.

The information read from RFID tag 802is displayed on the screen. For example, the withstand pressure of the syringe is displayed as "15.0 kgf/cm$^2$" at an icon. The iodine amount per unit weight required for visualization of the liver is displayed as "540 mgl/kg" at another icon. These icons are displayed over a human body image.

At this point, an injection amount icon indicating "A 100 ml" and "B 60 ml" may be displayed. This represents that the injection amount of contrast medium is 100 ml and the injection amount of physiological saline is 60 ml. An amount adjustment icon indicating "amount adjustment" may be displayed under the injection amount icon.

When the remaining chemical liquid amount in the syringe (for example, 70 ml) is smaller than the set injection amount (for example, 76 ml), amount adjustment icon 173d can be touched to set the remaining chemical liquid as the injection amount.

Next, the procedure of determining the injection amount of the contrast medium is described. In the present embodiment, the injection amount is calculated on the basis of (i) the weight of the patient, (ii) the iodine concentration of the contrast medium, and (iii) the iodine amount required per unit weight associated with the imaging region. Specifically, the calculation is performed assuming that the weight of the patient is 55 kg corresponding to an intermediate value of the range from 40 kg to 70 kg, by way of example. The total iodine amount to be injected into the patient is calculated on the basis of the weight and the required iodine amount, and the amount of the contrast medium to be injected is calculated on the basis of the total iodine amount and the iodine concentration of the contrast medium.

Although 55 kg is used as the representative value of the range from 40 kg to 70 kg in this example, the value may be changeable in initial setting. The boundaries of the weight range (for example, the range from 40 kg to 70 kg) may also be changeable in the initial setting.

In conventional chemical liquid injectors, determination of the amount of the contrast medium based on the weight of a patient typically requires users to manually input, for example weight information and contrast medium information, which complicates the work. According to the present invention, however, the weight information is input only by touching the single weight icon and the contrast medium information is read from RFID tag 802 to remarkably simplify the work.

Next, the calculation of the injection rate of contrast medium is described.

In the present embodiment, for example at the time of selection of the liver as the imaging region, the injection time period of "0:30 sec" is determined. The amount of the contrast medium to be injected calculated at the above step is divided by this injection time period to automatically determine the injection rate of the contrast medium.

The series of steps described above determines the injection time period [sec] of the contrast medium, the injection amount [ml] of the contrast medium, and the injection rate [ml/sec] of the contrast medium, thereby setting the injection conditions for the contrast medium. Since injection conditions for physiological saline can be determined with a known method, detailed description thereof is omitted.

The injection conditions for the contrast medium thus determined and the injection conditions for the physiological saline can be displayed as a thumbnail image schematically showing an injection graph in which the horizontal axis represents elapsed time and the vertical axis represents the injection rate. Alternatively, such a thumbnail image may be displayed before the input of the weight and the determination of the injection rate. For example, at the time of selection of one imaging region, a default weight range may be selected, the associated injection rate may be selected, and the associated thumbnail image may be automatically displayed.

The thumbnail image shows the graph in which the horizontal axis represents the elapsed time and the vertical axis represents the injection rate. In the graph, a condition image of horizontally long rectangular shape can be represented to show the injection rate and the injection amount of the chemical liquid. The condition image has a width corresponding to the injection time period, for example, and is displayed at a vertical position corresponding to the injection rate. The condition image includes the injection rate and the injection amount shown in numerical values.

Conventional apparatuses typically display, at the time of setting of injection conditions, information of the injection time period, injection rate, and injection amount simply as numerical values. A user may have difficulty in intuitively knowing what injection conditions are set. According to the apparatus of the present embodiment, however, the user can more intuitively understand the injection conditions through the thumbnail image.

It is possible that the set injection conditions may be displayed as the injection graph in the form of an enlarged version of the thumbnail image. In this case, however, the space to display various icons (for example, the injection time period icon, weight icons, and human body image) for setting the injection conditions is significantly limited. In contrast, with the configuration in which the thumbnail image is displayed, these icons and the human body image can be displayed without any change, and as required, those icons can be used to perform modifications easily.

The width and the vertical position of the condition image in the thumbnail image may or may not be changeable according to the set conditions.

By way of example, when the thumbnail image is touched, an enlarged version of the image may be displayed on touch panel 103. For example when physiological saline is set to be injected after the injection of the contrast medium, the enlarged version can show the condition image of the contrast medium and the subsequent condition image of the physiological saline as well as the withstand pressure of the syringe in an upper right portion of the graph. An upward arrow and a downward arrow may be displayed on the side of the condition image of the contrast medium and operated to modify the injection conditions. The injection conditions for the physiological saline may be modified similarly.

Instead of the above method, the injection rate may be changed, for example by the operator moving the condition image upward or downward while touching it. The numerical value in the condition image may be changeable by input of a numerical value with a numeric keypad. The condition image may be changed by pressing of predetermined "higher" and "lower" buttons.

Once the injection conditions are determined, touch panel 103 shows a screen for checking the finally set conditions. An image provided by enlarging the thumbnail image described above and schematically showing the graph in which the horizontal axis represents the elapsed time and the vertical axis represents the injection rate is displayed at the center of the screen. A horizontally long window is displayed over the image and includes information such as the weight of the patient, the iodine amount required per unit weight, and the injection time period. The injection amount icon is displayed on the right of the window and can be used to check the amounts of contrast medium (A) and physiological saline (B).

The window may display additional information such as the selected weight range. The window may display the iodine amount per unit weight or the iodine amount per unit weight and per time.

After the injection conditions are checked on the screen, the operator presses, for example, a "check" button displayed on touch panel 103 or check button 116a (see Fig. 3) on injection head 110 to make the injector ready for the start of chemical liquid injection. In this example, the chemical liquid injection is performed in the order of the contrast medium and then the physiological saline.

When the contrast medium injection is started, touch panel 103 shows a pressure graph in which the horizontal axis represents the elapsed time and the vertical axis represents the injection pressure. This graph shows the injection pressure of the chemical liquid during the injection in real time. The injection pressure is calculated with the result of the motor current measurement as described above.

The pressure graph can include not only the detected real-time pressure of the chemical liquid but also an ideal pattern (which may be displayed as a single line or may be displayed as a band having a certain width) which would be achieved from injection performed under the injection conditions.

The weight scale icon and the syringe icon described above are provided to switch the mode for setting the injection conditions. While the weight scale is selected, the setting is performed in a "weight input mode" (the setting procedure described above). This mode is used by default in the present embodiment. When the syringe icon is selected, the setting can be performed in the "flow rate mode". In the weight input mode, the injection time period (for example, 0:30 sec) has a higher priority, and the injection rate is calculated on the basis of the injection time period. In contrast, in the flow rate mode, an arbitrary injection rate can be set such as 1.5 ml/sec or 2.0 ml/sec. This mode is used, for example when the injection rate is an important factor in visualization.

The series of operations described above basically includes the following:

(a) the processing of displaying the images of the plurality of imaging regions on the display device;
(b) the processing of, when one of the imaging regions is selected, reading the injection time period associated with that imaging region;
(c) the processing of determining the amount of the contrast medium to be injected into the patient;
(d) the processing of determining the injection rate of the contrast medium based on the determined contrast medium amount and the injection time period;
(e) the processing of displaying the graph of the injection conditions showing the relationship between the injection time period and the injection rate as the thumbnail image on the display device; and
(f) the processing of, when the predetermined icon on the display device and/or the predetermined button on the injection head is pressed after the processing (e), entering the state ready for injection in accordance with the injection conditions. Each of the processing operations can be performed by console control circuit 152.

According to the series of processing operations as described above, when the injection conditions are set, the conditions are displayed as the thumbnail image on touch panel 103. The operator can check the settings by seeing the thumbnail image. Since the image is displayed as the relatively small thumbnail image instead of a large image, the other icons for setting the injection conditions can continue to be displayed at the original positions and can be used to perform modification easily.

The chemical liquid injector of the present embodiment allows the setting of the injection conditions through the simple steps including (i) mounting the syringe on the injection head, (ii) selecting the imaging region, and (iii) selecting the weight of the patient. To realize this simple setting, the associated injection time period of the contrast medium (for example, 0:30 sec) is preferably determined, for example at the time of selection of the imaging region. In addition, to omit the work of manually inputting the information of the contrast medium, the information of the iodine concentration of the contrast medium, the information of the amount of the contrast medium and the like are preferably read automatically from syringe RFID tag 802.

Although the weight of the patient may be input with a specific numerical value through the numeric keypad, the operation can be more simplified by selecting one of the plurality of weight icons as in the present embodiment.

In the apparatus of the present embodiment, the injection time period of the contrast medium is determined by the selected imaging region (0:30 sec in the above example), and the time period is basically fixed regardless of the weight of the patient. According to such a configuration, even when a single patient undergoes a plurality of examinations (in the same imaging region), or even when a plurality of patients undergo examinations (in the same imaging region), the same injection time period is used. This eliminates the need to adjust the timing of start of scanning in the CT apparatus or the like.

The present invention is not limited to the above, and the following is applicable.

For example, to use a syringe with no RFID tag 802 attached thereto, touch panel 103 can display a "product name" icon. This icon can be touched to show a pull-down list. One of products in the list can be selected to input the information of the product name, iodine amount and the like.

In the apparatus of the present embodiment, the predetermined injection time period (for example, 30 seconds, 45 seconds, or 60 seconds) is recorded for each of the imaging regions (for example, the tumor portion, liver, kidney, and blood vessel). Such data may be stored on the storage section of console control circuit 152 or may be stored in an external device and read into console control circuit 152 via a network.

A device for measuring the weight of the patient or a device having the weight information of the patient may be connected to console 101, and the weight information may be input from that device to automatically select the weight range of the patient. This can omit the step of selecting the weight.

Two or four or more weight icons may be displayed. Instead of selecting the weight range, a single weight value (for example, 60 kg) may be automatically selected by default.

In the procedure of starting the chemical liquid injection, the thumbnail image may continue to be displayed without displaying the enlarged condition image in the schematically shown graph as described above, and a predetermined button (an icon on the screen and/or a button on the injection head) may be pressed with the thumbnail image remaining displayed, thereby making the injector ready for the start of injection. For example, while the thumbnail image is displayed on the screen, the check button on touch panel 103 or check button 116a on injection head 110 may be operated. Then, the display of the check button on the screen is switched to "start OK" and the injector is ready for the start of injection.

Although the above description has been made of the human body image horizontally oriented by way of example, the present invention is not limited thereto, and a human body image vertically oriented may be displayed. The number of body parts included in the human body image is not limited to four but may be three or less or five or more.

Next, exemplary images displayed on touch panel 103 are described.

(Mode for Selection of Different Injection Method or Predetermined Editing)

The following description is made of a contrast medium injector in which an injection mode and an condition setting method (hereinafter referred to simply as an injection mode or the like) can be set or changed simply on console 101 to perform injection in various methods. The following description is also made of a contrast medium injector in which the injection mode or the like can be set according to the preference of each doctor even when a plurality of doctors use the injector.

Touch panel 103 can display a home screen. The home screen can be displayed, for example by operating home button 102b (see Fig. 2) provided for console 101. Alternatively, the home screen may be displayed by pressing a predetermined icon or the like on touch panel 103, or the home screen may be displayed automatically after the elapse of a predetermined time period (idle time period).

On the home screen, a plurality of icons are placed in matrix form (for example, including 3 rows and 4 columns). In this example, each icon has a generally square shape with rounded corners, but the icon may have a different shape such as a rectangle, polygon, or circle. Although the number of the icons displayed on the home screen is not particularly limited, too many icons may prevent a user from performing intuitive operation. For example, the number of the icons including 3 to 5 rows and 3 to 6 columns (that is, a 3 by 3 matrix to a 5 by 6 matrix) is preferable since a sufficient amount of information can be displayed without compromising the operability.

For example, four icons are displayed horizontally in line in the top row on the home screen. The icons are provided for a "child mode," "emergency mode," "plot injection mode," and "test bolus tracking (TBT) injection mode."

Four icons for selecting one of doctors 1 to 4 who should use this contrast medium injector are displayed in the middle row on the home screen. The number of the icons is not particularly limited, and for example, only one, two, or three icons may be displayed.

A plurality of icons for performing various functions of the chemical liquid injector are displayed in the bottom row on the home screen. For example, an icon for displaying the results of chemical liquid injection, an icon for performing protocol setting, an icon for performing various environment setting, and an icon for performing user (such as doctors) editing are displayed.

The arrangement of the icons on the home screen is preferably changeable as appropriate. Basically, rearrangement of the icons can be performed and the display/not display of each icon can be arbitrarily changed. However, some of the icons may be set to be displayed at all times on the screen (that is, they cannot be set to "not display"). For example, the icon for emergency mode can be set to be displayed at all times to appropriately perform chemical liquid injection for emergency.

Next, the function of each icon is described. In the following, the "emergency mode" is first described, and then the "child mode" or other modes are described.

(Emergency Mode)

For example when a specialized doctor is not present in medical care at night, the "emergency mode" is used in order to allow the injection of a contrast medium to be performed by another doctor. Once the emergency mode icon is selected on the home screen, the emergency mode is started to display an emergency mode screen on touch panel 103 after a predetermined operation (described later in detail).

A status bar is displayed in an upper portion on the screen and shows an ER display indicating that the emergency mode is being selected and some other icons.

The other icons include, for example, a "route icon" for performing operation for checking whether a route to a patient is normally established in chemical liquid injection, and an icon for performing a conventionally known "timing test." In addition, the status bar may show an identification mark indicating the product name and/or the manufacturer of a chemical liquid in the syringe. A start button is displayed on the right of the status bar.

A large window is displayed at the center of the screen and shows an injection condition image for the emergency mode and a guidance image for guiding the user in operation.

The injection condition image is a graph in which the vertical axis represents the injection rate and the horizontal axis represents the injection time period of the chemical liquid, and a condition bar image is displayed in the graph. Since it is important to quickly set injection conditions in the emergency mode, the condition bar image shows injection conditions including a preset injection rate and a preset injection time period (for example, a rate of 1.0 ml/sec and an amount of 100 ml). The injection conditions may or may not be changeable by a user.

The guidance image provides the user with information for guiding the user in which operation should be performed on injection head 110 and/or console 101. For example, the information indicating that an operation device of console 101 (switch unit 300 shown in Fig. 10A to Fig. 10G) should be operated is displayed as an image.

The emergency mode screen may include a button having an "i" mark. The guidance image may be displayed by operating this button. In an alternative embodiment, the guidance image may be displayed automatically after the elapse of a predetermined time without operating the i button.

On the emergency mode screen, a horizontally oriented information display bar is displayed between the window at the center of the screen and the status bar in the upper portion on the screen, and for example, shows the body image of a patient and the chemical liquid injection time period. By way of example, the body image of a patient is formed of a plurality of body parts, and a selected body part (described later in detail) may be highlighted. This allows the user to see which body part is being selected.

A chemical liquid amount display portion for displaying the chemical liquid amount in the syringe is displayed on the right of the horizontally oriented information display bar. For example when the syringe contains 150 ml of contrast medium, "150 ml" is displayed in the chemical liquid amount display portion.

Once the injection conditions and the like are checked on the emergency mode screen and the start button on the screen or the operation device (not shown) of console 101 is operated, the display on the screen is changed. On the changed screen, the start button includes characters changed to "check," and the guidance image includes a changed instruction. Specifically, the information indicating that check button 116a (see Fig. 2) on injection head 110 should be pressed is displayed as an image. Then, when check button 116a or the start button on the screen is operated, the chemical liquid injection on the emergency mode is started.

According to the configuration of the present embodiment, the operator can safely and quickly start the injection in the emergency mode by (1) selecting the emergency mode on the home screen, (2) checking the injection conditions, and then inputting "start OK" on the screen or console, and then (3) pressing the (exemplary) predetermined button on the injection head.

For performing the emergency mode, a selection screen for showing a plurality of preset injection conditions may be first displayed to allow selection of one of the plurality of injection condition candidates. Examples of the preset injection conditions include "head CTA," "chest/abdomen part CTA," "abdomen part acute abdominal condition (condition 1)," "abdomen part acute abdominal condition (condition 2)," and "abdomen part acute abdominal condition (condition 3)," each of which displays the imaging region, injection rate, and injection amount. According to such a configuration, the contrast medium can be injected appropriately by taking account of the region and patient characteristics even in the emergency mode. Once one of the plurality of injection conditions is selected, the emergency mode screen described above is displayed, for example.

In addition to the above example, the image may be displayed as follows. Specifically, when the emergency mode is selected on the home screen as described above, a selection screen for preset injection conditions is displayed. One of the injection condition candidates on the selection screen is selected to display the emergency mode screen described above. When the check button on the emergency mode screen or the check button on the injection head is pressed, the display of the icon on the screen is changed from "check" into "start OK." Then, the start button on the injection head or the switch on the switch unit is pressed to start the injection. The aspect of the display on the screen is changeable as required in this manner.

(Child Mode)

When the child mode icon is selected on the home screen, the child mode is started to display a child mode screen on touch panel 103. On the child mode screen, a vertically oriented human body image (by way of example) representing a child is displayed in a left portion. This human body image includes icons representing a plurality of selectable body parts (for example, a head part, a chest part, an abdomen part, and a leg part). The selected part can be highlighted.

In association with the selected body part, predetermined injection conditions previously set are displayed in a thumbnail image. For example, the thumbnail image shows the injection conditions indicating that 20 ml of contrast medium should be injected at a rate of 1.5 ml/sec and then 10 ml of physiological saline should be injected at a rate of 1.5 ml/sec. At the center of the child mode screen, a weight display portion for setting the weight of the child and an injection time period display portion showing the injection time period may be displayed.

According to the configuration of the present embodiment as described above, appropriate injection conditions for each patient can be set not only for adults but also for children. In the child mod, a plurality of preset injection condition candidates may be displayed as described in the emergency mode. For children, however, the setting of detailed injection conditions may be unnecessary in contrast to adults, so that such functions may be omitted to allow quicker and simpler setting.

The human body image for child is not limited to the vertical orientation but may be horizontally oriented. The child mode may be started, for example when an adapter for a small syringe (of 20 ml, for example) is mounted on the injection head. Specifically, when the adapter is mounted, switching may be automatically made to the screen of the human body image for child.

(Other Injection Modes)

When the plot injection icon or the test bolus tracking injection icon is selected on the home screen, the plot injection mode or the test bolus tracking injection mode is started. These modes are conventionally known and detailed description is omitted. According to the present embodiment, the various injection modes can be intuitively selected on the home screen to save the user some time and effort in setting the injection conditions.

The details of the icons displayed on the home screen do not limit the present invention in any way. It should be noted that icons for other condition setting methods (for example, a weight input mode, a lean body weight mode, a body surface area mode, and a blood amount mode) may be preferably displayed on the home screen according to the preference of the user.

Conventional methods can be used for setting those injection modes/conditions, and a lean body weight mode screen may be displayed for the lean body weight mode, for example. The screen preferably shows the patient's weight, height, chemical liquid information, and injection conditions, as well as an icon for selecting whether the patient is a man or a woman. The lean body weight mode screen has a window showing "LBW" indicating that the lean body weight mode is being selected and an iodine amount (540 mgl/Kg) per weight.

In each injection mode, a conventionally known and predetermined alarm may be issued for the user. For example, the alarm may be issued when the set injection rate, injection amount, or injection pressure is higher or lower than a predetermined reference value. In this case, the associated operation may be forcedly stopped in addition the issuance of the alarm.

(Doctor Selection Icon)

On the icons for doctor selection displayed on the home screen, a check mark can indicate which doctor is selected. When the doctor selection is not performed, the check mark may be put on doctor 1 by default.

Each doctor can set injection conditions according to the preference in advance. The doctor can set, in detail, (i) for example, which imaging region is displayed in response to selection of each body part in the imaging region selection mode, (ii) what injection time period and what injection rate are used, and (iii) which injection pattern is used.

According to this configuration, the doctor can use the condition setting mode according to the preference.

Other settings (for example, which icon is displayed on the home screen and in which arrangement the icons are displayed) may be arbitrarily changeable according to the preference of each doctor. For example, the arrangement of the icons on the home screen may be arbitrarily changed.

Some icons may be set such that they cannot be deleted from the home screen. For example, when the emergency mode is relatively important in any hospital, the emergency mode icon can be displayed at all times as described above and only the other icons can be changed into preferred icons.

(Icons for Performing Various Functions and the like)

The home screen can include the "protocol setting" icon. The "protocol setting" icon is used to set a new injection protocol or to change the existing injection protocol. The injection protocol is not particularly limited, but various types can be preferably set such as a protocol called variable injection in which the injection rate of the chemical liquid changes over time or a protocol for performing the injection of the contrast medium and then the injection of physiological saline.

In the protocol setting mode, guidance may be displayed to facilitate the new registration or the change of the injection protocol. An example of the guidance may be a setting screen which shows some items arranged in line to be set in the injection protocol. A specific numerical value range or the like may be input to each item to complete the registration or the change. The items may be arranged vertically or horizontally.

The home screen can include the "environment setting" icon. The "environment setting" icon is provided for performing various settings such as setting of date and time, setting of sound volume, setting for cooperation (association) with another medical equipment, and setting of leak detection.

The home screen can include the "user editing" icon. The "user editing" icon is provided for setting and registering a new doctor and changing an already registered item.

The home screen can include the "injection result" icon. The "injection result" icon is provided for displaying the results of the chemical liquid, and for example, may show an injection graph for a single chemical liquid injection or may show a list of data from a plurality of chemical liquid injections performed in the past (by way example, the date and time, injection pattern, injection rate, injection amount, injection pressure, and product name of contrast medium). For example, the graph showing the results of the chemical liquid injection (by way of example, including information indicating which chemical liquid was injected at which pressure, for how long) may be output to the outside and displayed together with a diagnostic image obtained by the imaging apparatus.

Touch panel 103 may display various screens such as a screen showing the state in which console 101 is connected to imaging apparatus 200. In this case, the predetermined injection protocol may be read from imaging apparatus 200 and set as the injection conditions. A message window on the screen displays, for example, the message of "protocol set from scanner."

In the contrast medium injector of the present embodiment, the region screen registered by default may be first displayed, and then the home screen may be displayed, for example in response to pressing of the home button (the hard key on the console). Instead of pressing the home button, a timer may be set such that the home screen is automatically displayed at a predetermined time.

[Other Functions of Injector]

(Self Check)

The chemical liquid injector may be configured to perform the following operations.

The chemical liquid injector according to one embodiment of the present invention may automatically perform a series of operations called "self check" when the main power (for example, power button 102c on console 101) is turned on. In the "self check," the piston driving mechanism of injection head 110, a predetermined switch or sensor and the like are operated to check whether or not the injector normally operates. For the piston driving mechanism, by way of example, the motor serving the driving source is actually rotated to check whether or not normal operation is performed. In this case, the rotation amount of the motor may be small. For example, the check may be performed with a rotation amount smaller than the rotation amount required for the chemical liquid injection (the main injection for visualization or predetermined pre-injection before the main injection).

For the switch or the sensor and the like, the check is performed, for example, to ensure the conducting state of those components or to see whether or not the output value falls within a predetermined range. Check may also be performed to see whether or not the connection between the devices is normally made.

The results of the self check may be displayed sequentially on the display. For example, items including "rate," "amount," "pressure," "STOP," "switch," and "connection" can be checked sequentially.

According to the configuration, the use of the injector can be started after the self check performed at the start of the injector ensures the normal functions of the injector, thereby preventing any troubles due to a fault of the injector or a poor connection.

(Guidance Function in Protocol Setting)

Although the description has been made of the display of the predetermined guidance image in the emergency mode in response to the pressing of the "i" button, such a guidance image may be displayed, for example during the procedure of setting the injection protocol (that is, the injection conditions). For example when some items need to be input for setting the injection protocol, the completion of input of one item may be followed by guidance display showing the next item to be input. Such guidance may be automatically displayed or may be displayed when the user performs a predetermined input.

(TBT Mode)

The use of a test injection method or a bolus tracking method, for example, has conventionally been proposed in order to determine the imaging timing in the imaging apparatus. The details of the bolus tracking method are described, for example in WO2011/136218 filed earlier by the present applicant, and detailed description thereof is omitted. The outlines are as follows. In the bolus tracking method, the imaging apparatus monitors an increase in CT value of a predetermined region of interest during the injection of the contrast medium, the CT value reaching a predetermined value is used as a trigger, and after a predetermined delay time period, main scan is started. The injector of the present embodiment is configured as described above such that the icon for test bolus tracking injection mode is displayed on the home screen and is touched to enter the test bolus tracking mode.

In the bolus tracking test, as described WO2011/136218, a patient holds his breath to stabilize the heart rate during the delay time period after the first injection of the contrast medium. In such a series of injection operations, for example, the touch panel on the console may display a window of injection conditions for the contrast medium and physiological saline and a window of time line for the bolus tracking. The window of time-line has an interval (delay time period) section in which character information may be displayed such as "delay," "breath held," and "heart rate stabilized." The number of seconds (5 seconds for each in this case) may be displayed in combination with each character information.

When the injection of the chemical liquid is started, the current injection status may be displayed. Specifically, a status bar in an upper portion on the screen may display a plurality of indicators showing which process is being performed. A first indicator shows that the "test bolus" injection is being performed, a second indicator shows that the operation is in the interval, and a third indicator shows that the "bolus" injection is being performed. The indicator showing the currently performed operation may be highlighted.

(Data Writing to External Recording Medium)

In the injector of one embodiment of the present invention, after the injection is completed, the information of the injection results and the like may be writable to an external storage medium. The storage medium may be a memory medium insertable into a predetermined slot (not shown) of the console. The written information is not particularly limited, and any information relating to the injection results may be written. The data may be written in CSV format so as to facilitate the use of the injection results in another personal computer or the like. However, the format of the data is not limited thereto, and various formats can be used. The writing of the injection results is not limited to the storage medium such as the memory, but the injection results may be sent to a predetermined system (for example, a hospital management system) over a network and saved in an internal storage apparatus, for example.

(Display of Information on Head Display)

The present invention may also include the following functions. For example, a sub display (also called a head display) different from touch panel 103 of console 101 may be included. The sub display may be integral with the injection head or may be attached to the mechanism for supporting the injection head (for example, the stand or the articulated arm for ceiling mounting). The sub display may show, for example, a graph representing pressures during injection. Specifically, the pressure graph may be displayed in real time on at least one of the main display and the sub display by the console and the head operated in synchronization. For example, a graph representing pressures during a "route check" may be displayed on the sub display. The "route check" is mainly performed to check whether the chemical liquid route is normally established (for example, whether an injection needle is correctly inserted, or whether a kink is formed in the tube). The ability to check the pressure graph during the route check on the sub display near the patient is advantageous in finding incorrect insertion of the injection needle or unkinking the tube.

The injection rate in the chemical liquid injection for the route check may be automatically set to be the same as the injection rate in the main injection. Alternatively, the injection rate for the route check may be set independently of the injection rate in the main injection.

(Preset Injection Protocol)

The injection protocol may be preset. The preset of the injection protocol can simplify the injection protocol setting procedure. In the preset of the injection protocol, only one injection protocol can be preset, but a plurality of injection protocols are preferably preset to allow the operator to select one of them.

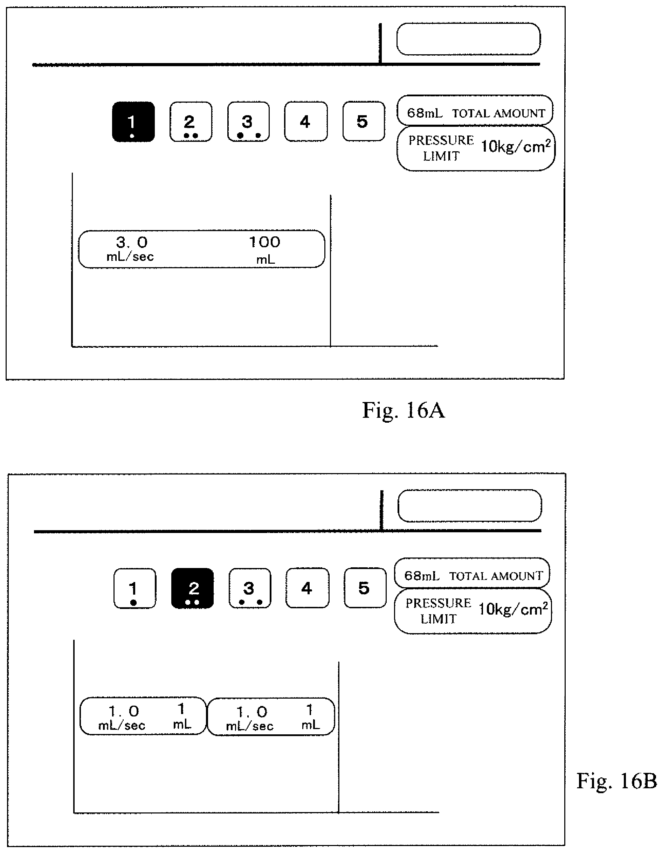

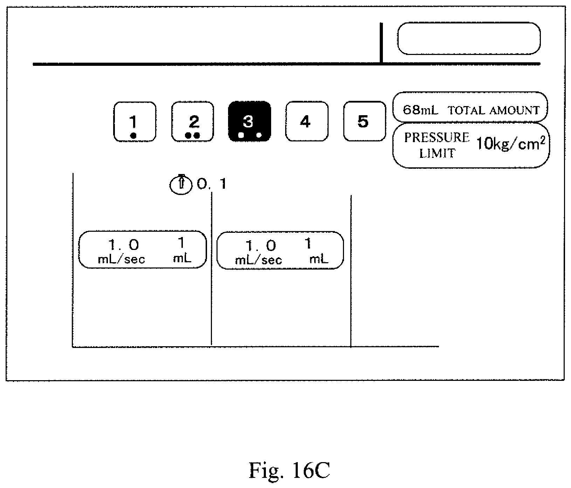

Next, description is made of exemplary screens displayed on the touch panel when the plurality of injection protocols can be preset with reference to Fig. 16A to Fig. 16C.

Fig. 16A to Fig. 16C show examples displayed when icons having preset numbers 1 to 3 are selected, respectively. The preset number can be selected, for example, by touching an icon displayed on the screen. The selected icon can be visually distinguished from the icons not selected, by its color changed from that of the other icons.

When the preset number is selected, the associated injection graph, amount of chemical liquid filled in the syringe, pressure limit and the like are displayed. As shown, the injection graph may schematically show the injection rate and the injection amount in numerical values. In the example shown, the injection graph is an injection graph in which the vertical axis represents the injection rate and the horizontal axis represents time. The vertical axis and the horizontal axis in the injection graph preferably have scales as appropriate. When the operator checks the injection graph and the like and determines that the selected injection protocol should be used, the operator can perform an additional operation of re-touching the selected icon or touching a "check" icon displayed on the screen to confirm the selected injection protocol.

To give the operator an outline of the injection protocol associated with the preset number without displaying the injection graph, a dot may be displayed in a lower portion of the icon showing the preset number. In the example shown, the dot displayed in the lower portion of the injection icon represents the number of phases and the presence or absence of the interval. Specifically, the number of dots represents the number of injection phases, and when the injection protocol includes a plurality of injection phases, dots at a narrower interval represent closely spaced phases, and dots at a wider interval represent phases with a certain interval present between them. An icon having no dot indicates that no injection protocol is preset. The above is an example of the display, and any other display is possible.

(Example of Injection Pressure Display in Dual-Syringe Type)

In the chemical liquid injector, for example as shown in Fig. 14, two or more syringes can be mounted simultaneously, and the chemical liquids in the mounted syringes can be injected individually or simultaneously. When the chemical liquids in the plurality of syringes are injected simultaneously, injection pressure graphs for all the chemical liquids are preferably displayed on the display device (for example, the touch panel of the console and/or another display, if any). However, only one injection pressure graph may be displayed, for example due to a limited area on the screen available for the display of the injection pressure graph. Next, description is made of how display is performed when the injection pressure reaches the limit pressure to temporarily stop the motor during injection of the chemical liquid through the operation of first and second piston driving mechanisms in the case described above.

First, during the normal injection operation, an injection pressure graph of data from an arbitrary one of the first and second piston driving mechanisms can be displayed. The injection pressure graph to be displayed can be an injection graph from a preset piston driving mechanism or can be set arbitrarily by the operator. For example, the injection graph including a higher injection pressure or a lower injection pressure may be displayed. In this case, the injection graph with a higher injection pressure is preferably displayed. Alternatively, the average value of both injection pressures may be calculated and the resulting average injection pressure graph may be displayed.

When the injection pressure of the chemical liquid with the first or second piston driving mechanism reaches the limit pressure, and the driving of the motor for that piston driving mechanism is stopped, either the injection pressure graph from the stopped piston driving mechanism or the injection pressure graph from the normally operating piston driving mechanism may be displayed. To inform the operator of the occurrence of abnormality, the injection pressure graph from the stopped piston driving mechanism is preferably displayed. Specifically, when the injection pressure graph displayed before the stop is the injection pressure from the stopped piston driving mechanism, the display of the injection pressure graph is continued, and when not, the display is switched to the injection pressure graph from the stopped piston driving mechanism.

After the start of the injection, the injection pressure data measured on the basis of the motor current can be accumulated in the memory (for example, the RAM of head control circuit 158). For displaying the injection pressure graph from the stopped piston driving mechanism, the accumulated injection pressure data after the injection start can be displayed to allow the operator to see the process of pressure increase.

When both the first and second piston driving mechanisms are stopped, the injection pressure from either piston driving mechanism may be displayed similarly to the above case. The piston driving mechanism for which the injection pressure graph is displayed may be switched by the operator. The case where both the piston driving mechanisms are stopped includes the case where both are stopped simultaneously and the case where one of them is stopped during the stop of the other and consequently both of them are stopped.

As described above, the injection pressure graph from either piston driving mechanism may be displayed. However, when the contrast medium is injected as the chemical liquid with one of the piston driving mechanisms and physiological saline is injected with the other piston driving mechanism, the injection pressure graph from the piston driving mechanism used to inject the contrast medium is preferably displayed with a higher priority.

(Application as Filling device)

The chemical liquid injector can serve as a chemical liquid filling device when an empty syringe with no chemical liquid filled and having a piston located at the foremost position is mounted thereon, and before or after the mounting, the empty syringe is brought into fluid communication with an external chemical liquid container (in an arbitrary form such as a bag or a bottle) containing the chemical liquid through a tube or the like, and in this state, the piston driving mechanism is operated to move the piston rearward. The empty syringe can have the same configuration as that described with reference to Fig. 11 and Fig. 12.

When the chemical liquid injector is used as the chemical liquid filling device, presser 112 (see Fig. 3) includes a piston flange holding structure like a plurality of hooks which can not only press the piston of the syringe but also hold and move a flange of the piston rearward. Thus, moving presser 112 rearward by the operation of the piston driving mechanism enables the piston to be moved rearward.

The rearward movement of presser 112 can be performed, for example by the operator pressing rearward button 116e (see Fig. 3) provided for injection head 110. The operator can fill an arbitrary amount of chemical liquid into the syringe.

Alternatively, the home screen described above may include an additional icon for "filling mode" such that the icon can be touched (selected) to switch to the filling mode. In the filling mode, a filling amount is set and the operation of the piston driving mechanism is controlled to fill the set amount of chemical liquid into the syringe. The setting of the filling amount in the filling mode, the setting of a screen displayed on touch panel 103, and the control of operation of piston driving mechanism 161 (see Fig. 5) as described below can be implemented as a computer program which is executed by console control circuit 152 (see Fig. 5) and head control circuit 158 (see Fig. 5).

In the following, an example of processing after the switching to the filling mode is described.

In the filling mode, at least the filling amount may be set, and a filling rate can be preset appropriately not to affect the operation of the piston driving mechanism. The filling amount of chemical liquid can be calculated from the internal diameter of the mounted syringe and the rearward movement distance of presser 112. Once the internal diameter or the model number of the syringe (the internal diameter of the syringe can be specified from the model number) and the filling amount are input through the input device such as touch panel 103, console control circuit 152 calculates the rearward movement distance of presser 12 based on the input values and transmits the calculated data to head control circuit 158. Head control circuit 158 drives piston driving mechanism 161 based on the data transmitted from console control circuit 152. This causes the syringe to be filled with the input amount of chemical liquid.

The empty syringe to be filled with the chemical liquid preferably has RFID tag 802 as described above attached thereto. Various types of data relating to the syringe including the internal diameter of the syringe are recorded on RFID tag 802, and the data recorded on the RFID tag is acquired by RFID module 166 and transmitted to console control circuit 152, which can eliminate the need for the operator to input the internal diameter of the syringe.

In addition, the information relating to the chemical liquid such as the filling amount, type, manufacturer, product number, viscosity of the chemical liquid, the iodine concentration when the chemical liquid is a contrast medium, expiration date, and filling date and time can be written to RFID tag 802 by RFID module 166 after the chemical liquid is filled. An RFID tag similar to RFID tag 802 on the syringe can also be attached to the chemical liquid container including the chemical liquid to be filled, and the information relating to the chemical liquid except the information relating to the filling amount can be recorded on that RFID tag, so that the information can be used as the information to be written to RFID tag 802 on the syringe. To use the information written on the RFID tag attached to the chemical liquid container as the information to be written to RFID tag 802 attached to the syringe, an RFID module (RFID reader) different from RFID module 166 provided for injection head 110 can be connected to injection head 110 and used to read the information from the RFID tag attached to the chemical liquid container, the read information can be temporarily held on head control circuit 158, and the held information can be written to RFID tag 802 by RFID module 166. The writing of the information can be performed simultaneously with or separately from the writing of the information relating to the filling amount and the filling date and time.

Although the filling amount of chemical liquid can be set arbitrarily by the operator, for example in accordance with the capacity of the syringe, the same amount as the injection amount into the patient is preferable due to economical use of the chemical liquid. For setting the filling amount of chemical liquid to be the same as the injection amount into the patient, the injection amount may be calculated in console control circuit 152, for example in accordance with the procedure described above, based on the weight of the patient, the imaging region, and the information relating to the chemical liquid acquired from the RFID tag attached to the chemical liquid container, and the calculated injection amount may be used as the filling amount and set in console control circuit 152.

The information relating to the filled chemical liquid is written to RFID tag 802 as described above. For example when the syringe filled with the chemical liquid is not used immediately but removed once from injection head 110 and stored temporarily, the syringe can be handled similarly to the pre-filled syringe having the RFID tag since RFID tag 802 has the necessary information written thereon.

During the filling of the chemical liquid, the filling status of the chemical liquid is preferably displayed on touch panel 103 to allow the operator to visually recognize that the filling is being performed. Examples of the display in this case include an image representing the chemical liquid being filled into the syringe by animation, an image showing a gradually increased numerical value indicating the filling amount of chemical liquid in association with the operation of piston driving mechanism 161, or a combination of these images.

To detect mixture of air bubbles into the syringe during the filling of the chemical liquid, an air bubble detection sensor is preferably placed on a tube which connects the chemical liquid container with the syringe. The air bubble detection sensor may be any detection sensor of an optic, ultrasonic, or electrostatic type that can detect whether or not any air bubbles are present in the tube. Any of those types detects the presence or absence of air bubbles in the tube by detecting a characteristic change when air bubbles are mixed, for example a change in optical characteristics such as refractivity, reflectivity, and transmissivity for the optical type, a change in resonance characteristics for the ultrasonic type, and a change in electrostatic capacitance for the electrostatic type.

(Data Carrier)

Although the above embodiment has shown the example where the data carrier for the syringe and the chemical liquid container is the RFID tag, the present invention can employ various types of data carriers such as a barcode and a two-dimensional code in addition to the RFID tag. When the data carrier other than the RFID tag is used, a reader adapted for the data carrier is used.

Since the barcode and the two-dimensional code do not support additional writing or rewriting of data, the barcode or the two-dimensional code can be used as the data carrier only in applications where data does not need to be additionally written or rewritten. Since the barcode can record only a small amount of data, it can be used as an identification code for distinguishing each syringe or the like instead of data recording. Various data about the chemical liquid or the like can be stored in a database inside or outside the chemical liquid injection system in association with individual syringes or the like, and when the identification code is read by a reader, the data associated with the identification code can be acquired from the database.

(Example of Screen Display When Mounting of Syringe with Data Carrier is Detected)

The reader (reader/writer) provided for the injection head is installed at a position where the reader can automatically read data recorded on the data carrier when the syringe having the data carrier attached thereto is mounted on the injection head.

When the syringe having the data carrier attached thereto is mounted on the injection head and the reader is activated for reading the data recorded on the data carrier, the head control circuit can transmit a data carrier detection start signal indicating that the reader starts the detection of the data from the data carrier to the console control circuit. The console control circuit can display an image during the data carrier detection on the console or another display device.

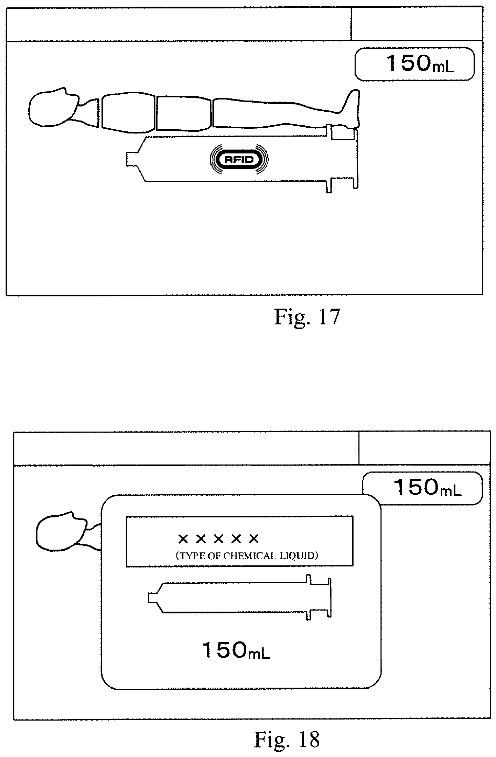

Fig. 17 shows a screen during data carrier detection displayed when the data carrier is the RFID tag, by way of example. In the example shown in Fig. 17, an image schematically showing the syringe is displayed as the image during data carrier detection at the center of the screen together with a mark representing the RFID tag. Preferably, a seal having the same mark as that mark is attached to the syringe, or the same mark as that mark is printed on the syringe. This allows the user to immediately see that the syringe with the RFID tag is mounted on the injection head and that the data is being read. As shown in Fig. 17, the image schematically showing the syringe can be displayed to overlap with the originally displayed image.

Alternatively, after the reading of the data from the data carrier, the console control circuit may display information relating to at least some of the read data on the console or another display device based on data transmitted from the head control circuit.

Fig. 18 shows an example of a displayed screen after the data reading. In the example shown in Fig. 18, a syringe information image is displayed at the center of the screen, including information representing the type of the chemical liquid, an image schematically showing the syringe, and the amount of chemical liquid filled in the syringe. As shown in Fig. 18, the syringe information image can be displayed to overlap with the originally displayed image. The information representing the chemical liquid may be at least one of the manufacturer, type, and product name of the chemical liquid. The display of at least some of the information read from the data carrier allows an immediate check of whether or not the mounted syringe is a syringe filled with the chemical liquid which should be injected in the subsequent operation.

Fig. 17 and Fig. 18 show examples of the screen display, and the displayed images are not limited thereto and can be changed appropriately as desired. The displayed image may be only the image during detection as shown in Fig. 17, may be only the image displaying the read information as shown in Fig. 18, or may be both of them. When both are displayed, the image during data carrier detection as shown in Fig. 17 can be displayed during the detection, for example over 1.5 seconds, and after the data reading is normally completed, the syringe information image as shown in Fig. 18 can be displayed, for example over three seconds, and then the syringe information image can be closed.

## DESCRIPTION OF REFERENCE NUMERALS

| | |
|---|---|
| 100 | CHEMICAL LIQUID INJECTOR |
| 101 | CONSOLE |
| 103 | TOUCH PANEL |
| 110 | INJECTION HEAD |
| 112 | PRESSER |
| 152 | CONSOLE CONTROL CIRCUIT |
| 153, 155 | WIRELESS COMMUNICATION MODULE |
| 158 | HEAD CONTROL CIRCUIT |
| 162 | MOTOR |
| 163 | MOTOR CURRENT MEASURING CIRCUIT |
| 164 | RFID CONTROL CIRCUIT |
| 165 | ANTENNA |
| 166 | RFID MODULE |
| 130 | POWER-SUPPLY BOX |
| 200 | IMAGING DIAGNOSTIC APPARATUS |
| 600 | ADAPTER |
| 800 | SYRINGE |
| 802 | RFID TAG |

## Claims

1. A chemical liquid injector for injecting a chemical liquid filled in a syringe (800) having a cylinder and a piston, comprising:

   a piston driving mechanism (112, 113) driven by a motor for moving the piston of the syringe (800) into and out of the cylinder;
   a motor current measuring circuit (163) configured to measure a motor current, the motor current being a current flowing through the motor;
   a display device (103); and
   an injector control circuit (152, 158) configured to control operation of the motor such that the chemical liquid in the syringe (800) is injected in accordance with an injection protocol for the chemical liquid, to calculate an injection pressure of the chemical liquid by using the result of the motor current measurement by the motor current measuring circuit (163),
   wherein the injector control circuit (152, 158) is configured to display the calculated injection pressure on the display device (103) while a chemical liquid injection operation is performed by driving the motor, to temporarily stop the operation of the motor when the calculated injection pressure reaches a preset limit pressure during the injection of the chemical liquid,
   **characterized in that** the injector control circuit (152, 158) is configured to display the preset limit pressure as the injection pressure on the display device (103) instead of the calculated injection pressure during the stop of the operation of the motor due to the injection pressure reaching the preset limit pressure.

2. The chemical liquid injector according to claim 1, wherein the motor current measuring circuit (163) is configured to measure the motor current at regular time intervals, and the injector control circuit (152, 158) is configured to calculate the injection pressure each time the motor current is measured.

3. The chemical liquid injector according to claim 1, wherein the motor current measuring circuit (163) is configured to measure the motor current at regular time intervals, and the injector control circuit (152, 158) is configured to calculate the injection pressure by using an average value of measurement values from a plurality of preceding measurements.

4. The chemical liquid injector according to any one of claims 1 to 3, wherein the injector control circuit (152, 158) is configured to display a graph representing a change in the injection pressure over time on the display device (103) during the injection of the chemical liquid.

5. The chemical liquid injector according to any one of claims 1 to 4, wherein the injector control circuit (152, 158) is configure to increase the number of revolutions of the motor over time at start-up of the motor such that an injection rate of the chemical liquid reaches an injection rate specified according to the injection protocol at a predetermined time which is set in accordance with a characteristics of the motor and the injection rate such that inrush current through the motor does not affect the calculation of the injection pressure.

6. The chemical liquid injector according to any one of claims 1 to 5, wherein the chemical liquid injector includes a console (101) including the display device (103), and an injection head (110) including the piston driving mechanism (112, 113) and formed as a unit independent of the console (101).

7. The chemical liquid injector according to claim 6, further comprising a wireless communication module (153, 155) for transmitting and receiving a signal and data between the console (101) and the injection head (110).

8. The chemical liquid injector according to any one of claims 1 to 5, wherein the syringe (800) includes an RFID tag (802), information relating to the filled chemical liquid and the syringe (800) being recorded on the RFID tag (802), and
the system further comprises a reader (166) configured to read the information from the RFID tag (802).

9. The chemical liquid injector according to claim 8, further comprising a holding mechanism (111) for removably holding the syringe (800),
wherein the reader (166) includes an antenna (165) located at a position facing the RFID tag (802) while the syringe (800) is held in the holding mechanism (111).

10. The chemical liquid injector according to claim 9, wherein the antenna (165) includes a flexible printed circuit having a pattern made of conductor formed thereon and is bent in an arc shape concentric with the syringe (800).

11. The chemical liquid injector according to claim 9 or 10, wherein the antenna (165) includes a ferrite sheet (165a) on a surface opposite to a surface facing the RFID tag (802).

12. The chemical liquid injector according to any one of claims 1 to 11, further comprising the syringe (800).

13. The chemical liquid injector according to claim 12, wherein the syringe (800) is a pre-filled syringe.

14. The chemical liquid injector according to claim 12 or 13, wherein the syringe (800) includes a cylinder flange (801; 501a) formed at a trailing end of the cylinder (501), and the cylinder flange (801; 501a) includes a notch portion (505) on an outer peripheral portion.

15. The chemical liquid injector according to claim 1, further comprising an empty syringe filled with no chemical liquid,
wherein the injector control circuit (152, 158) is configured to allow filling of the empty syringe with a chemical liquid from an external chemical liquid container by operating the piston driving mechanism (112, 113) such that the piston is moved rearward relative to the cylinder.

16. The chemical liquid injector according to claim 15, wherein the empty syringe includes an RFID tag, and the chemical liquid injector further comprises a writer for writing information relating to the filled chemical liquid to the RFID tag provided for the empty syringe.

**Patentansprüche**

1. Ein Injektor für chemische Flüssigkeit zum Injizieren einer chemischen Flüssigkeit in einer Spritze (800) mit einem Zylinder und einem Kolben, aufweisend:

einen Kolbenantriebsmechanismus (112, 113), der von einem Motor zum Bewegen des Kolbens der Spritze (800) in den und aus dem Zylinder angetrieben wird;
einen Motorstrommessschaltkreis (163), der konfiguriert ist, um einen Motorstrom zu messen, wobei der Motorstrom ein durch den Motor fließender Strom ist;
eine Anzeigevorrichtung (103); und
einen Injektorsteuerschaltkreis (152, 158), der konfiguriert ist, um den Betrieb des Motors derart zu kontrollieren, dass die chemische Flüssigkeit in der Spritze (800) in Übereinstimmung mit einem Injektionsprotokoll für die chemische Flüssigkeit injiziert wird, um einen Injektionsdruck der chemischen Flüssigkeit zu berechnen, indem das Ergebnis der Motorstrommessung durch den Motorstrommessschaltkreis (163) benutzt wird,

wobei der Injektorsteuerschaltkreis (152, 158) derart konfiguriert ist, dass der berechnete Injektionsdruck auf der Anzeigevorrichtung (103) angezeigt wird, während der Vorgang der Injektion einer chemischen Flüssigkeit durch den Betrieb des Motors ausgeführt wird, um den Betrieb des Motors zeitweise zu stoppen, wenn der berechnete Injektionsdruck während der Injektion der chemischen Flüssigkeit einen vorgegebenen Grenzdruck erreicht,

**dadurch gekennzeichnet, dass**

der Injektorsteuerschaltkreis (152, 158) derart konfiguriert ist, dass der vorgegebene Grenzdruck als der Injektionsdruck auf der Anzeigevorrichtung (103) anstelle des berechneten Injektionsdrucks während des Stoppens des Betriebs des Motors aufgrund dessen, dass der Injektionsdruck den vorgegebenen Grenzdruck erreicht, angezeigt wird.

2. Der Injektor für chemische Flüssigkeit nach Anspruch 1, wobei der Motorstrommesschaltkreis (163) derart konfiguriert ist, dass der Motorstrom in regulären Zeitintervallen gemessen wird, und der Injektorsteuerschaltkreis (152, 158) derart konfiguriert ist, dass der Injektionsdruck immer dann berechnet wird, wenn der Motorstrom gemessen wird.

3. Der Injektor für chemische Flüssigkeit nach Anspruch 1, wobei der Motorstrommesschaltkreis (163) derart konfiguriert ist, dass der Motorstrom in regulären Zeitintervallen gemessen wird, und der Injektorsteuerschaltkreis (152, 158) derart konfiguriert ist, dass der Injektionsdruck aus einem Mittelwert der Messwerte von einer Vielzahl von vorausgehenden Messungen berechnet wird.

4. Der Injektor für chemische Flüssigkeit nach irgendeinem der Ansprüche 1 bis 3, wobei der Injektorsteuerschaltkreis (152, 158) derart konfiguriert ist, dass während der Injektion der chemischen Flüssigkeit ein Graf auf der Anzeigevorrichtung angezeigt wird, welcher eine Veränderung des Injektionsdrucks über die Zeit darstellt.

5. Der Injektor für chemische Flüssigkeit nach irgendeinem der Ansprüche 1 bis 4, wobei der Injektorsteuerschaltkreis (152, 158) derart konfiguriert ist, dass die Anzahl der Motorumdrehungen pro Zeit zum Zeitpunkt des Startens des Motors derart erhöht wird, dass eine Injektionsrate der chemischen Flüssigkeit eine Injektionsrate erreicht, die entsprechend dem Injektionsprotokoll zu einer vorbestimmten Zeit, die in Übereinstimmung mit einer charakteristischen Eigenschaft des Motors festgesetzt ist, spezifiziert ist, und die Injektionsrate, dass der Einschaltstrom durch den Motor die Berechnung des Injektionsdrucks nicht beeinflusst.

6. Der Injektor für chemische Flüssigkeit nach irgendeinem der Ansprüche 1 bis 5, wobei der Injektor für chemische Flüssigkeit eine die Anzeigevorrichtung umfassende Konsole (101) und einen Injektionskopf umfasst, welcher den Kolbenantriebsmechanismus (112, 113) umfasst und als eine von der Konsole unabhängige Einheit ausgebildet ist.

7. Der Injektor für chemische Flüssigkeit nach Anspruch 6 ferner aufweisend ein drahtloses Kommunikationsmodul (153, 155) zum Senden und Empfangen eines Signals und Daten zwischen der Konsole (101) und dem Injektionskopf (110).

8. Der Injektor für chemische Flüssigkeit nach irgendeinem der Ansprüche 1 bis 5, wobei die Spritze (800) einen RFID-Tag (802) umfasst, wobei auf dem RFID-Tag (802) Informationen betreffend der abgefüllten chemischen Flüssigkeit und der Spritze (800) abgespeichert sind, und

das System ferner ein Lesegerät (166) aufweist, das konfiguriert ist, um die Informationen von dem RFID-Tag (802) zu lesen.

9. Der Injektor für chemische Flüssigkeit nach Anspruch 8 ferner aufweisend einen Halterungsmechanismus (11) zum abnehmbaren Halten der Spritze (800), wobei

das Lesegerät (166) eine Antenne (105) umfasst, die in einer Position angeordnet ist, die dem RFID-Tag (802) zugewandt ist, während die Spritze (800) in dem Halterungsmechanismus (111) gehalten wird.

10. Der Injektor für chemische Flüssigkeit nach Anspruch 9, wobei die Antenne einen flexiblen gedruckten Schaltkreis mit einem Muster umfasst, das aus einem darauf ausgebildeten Leiter besteht, und in eine Bogenform gebogen ist, die mit der Spritze (800) konzentrisch ist.

11. Der Injektor für chemische Flüssigkeit nach Anspruch 9 oder 10, wobei die Antenne (165) eine Ferritschicht (165a) auf einer Oberfläche umfasst, welche einer dem RFID-Tag (802) zugewandten Oberfläche gegenüberliegt.

12. Der Injektor für chemische Flüssigkeit nach irgendeinem der Ansprüche 1 bis 11 ferner aufweisend eine Spritze (800).

13. Der Injektor für chemische Flüssigkeit nach Anspruch 12, wobei die Spritze (800) eine vorgefüllte Spritze ist.

14. Der Injektor für chemische Flüssigkeit nach Anspruch 12 oder 13, wobei die Spritze (800) einen Zylinderflansch (801; 501a) umfasst, welcher an einem nachfolgenden Ende des Zylinders (501) ausgebildet ist, und der Zylinderflansch (801; 501a) einen Kerbenabschnitt (505) an einem äußeren peripheren Abschnitt aufweist.

15. Der Injektor für chemische Flüssigkeit nach Anspruch 1 ferner aufweisend eine leere Spritze, die nicht mit chemischer Flüssigkeit gefüllt ist, wobei der Injektorsteuerschaltkreis (152, 158) derart konfiguriert ist, dass es möglich ist, die leere Spritze mit einer chemischen Flüssigkeit von einem externen Behälter für chemische Flüssigkeit dadurch zu füllen, dass der Kolbenantriebsmechanismus (112, 113) derart betrieben wird, dass der Kolben relativ zum Zylinder zurück bewegt wird.

16. Der Injektor für chemische Flüssigkeit nach Anspruch 15, wobei die leere Spritze einen RFID-Tag umfasst, und
der Injektor für chemische Flüssigkeit ferner einen Schreiber zum Schreiben von Informationen betreffend die abgefüllte chemische Flüssigkeit in den RFID-Tag, der für die leere Spritze vorgesehen ist, aufweist.

**Revendications**

1. Dispositif d'injection de produit chimique liquide pour injecter un produit chimique liquide remplissant une seringue (800) ayant un cylindre et un piston, comprenant :

un mécanisme d'entraînement de piston (112, 113) entraîné par un moteur pour déplacer le piston de la seringue (800) dans et hors du cylindre ;
un circuit de mesure de courant de moteur (163) configuré pour mesurer un courant de moteur, le courant de moteur étant un courant circulant à travers le moteur ;
un dispositif d'affichage (103) ; et
un circuit de commande de dispositif d'injection (152, 158) configuré pour commander un fonctionnement du moteur de sorte que le produit chimique liquide dans la seringue (800) est injecté conformément à un protocole d'injection du produit chimique liquide, pour calculer une pression d'injection du produit chimique liquide en utilisant le résultat de la mesure du courant de moteur par le circuit de mesure de courant de moteur (163),
dans lequel le circuit de commande du dispositif d'injection (152, 158) est configuré pour afficher la pression d'injection calculée sur le dispositif d'affichage (103) alors qu'une opération d'injection de produit chimique liquide est effectuée par l'entraînement du moteur, pour arrêter temporairement le fonctionnement du moteur lorsque la pression d'injection calculée atteint une pression limite prédéfinie pendant l'injection du produit chimique liquide,
**caractérisé en ce que** le circuit de commande du dispositif d'injection (152, 158) est configuré pour afficher la pression limite prédéfinie en tant que pression d'injection sur le dispositif d'affichage (103) plutôt que la pression d'injection calculée pendant l'arrêt du fonctionnement du moteur lorsque la pression d'injection atteint la pression limite prédéfinie.

2. Dispositif d'injection de produit chimique liquide selon la revendication 1, dans lequel le circuit de mesure de courant de moteur (163) est configuré pour mesurer le courant de moteur à des intervalles de temps réguliers, et le circuit de commande du dispositif d'injection (152, 158) est configuré pour calculer la pression d'injection à chaque fois que le courant de moteur est mesuré.

3. Dispositif d'injection de produit chimique liquide selon la revendication 1, dans lequel le circuit de mesure de courant de moteur (163) est configuré pour mesurer le courant de moteur à des intervalles de temps réguliers, et le circuit de commande du dispositif d'injection (152, 158) est configuré pour calculer la pression d'injection en utilisant une valeur moyenne des valeurs de mesure provenant d'une pluralité de mesures précédentes.

4. Dispositif d'injection de produit chimique liquide selon l'une quelconque des revendications 1 à 3, dans lequel le circuit de commande de dispositif d'injection (152, 158) est configuré pour afficher un graphique représentant un changement de la pression d'injection dans le temps sur le dispositif d'affichage (103) pendant l'injection du produit chimique liquide.

5. Dispositif d'injection de produit chimique liquide selon l'une quelconque des revendications 1 à 4, dans lequel le circuit de commande du dispositif d'injection (152, 158) est configuré pour augmenter le nombre de tours du moteur dans le temps au démarrage du moteur de sorte qu'une vitesse d'injection du produit chimique liquide atteint une vitesse d'injection spécifiée conformément au protocole d'injection à un moment prédéterminé qui est fixé en fonction d'une caractéristique du moteur et de la vitesse d'injection de sorte qu'un courant d'appel à travers le moteur n'affecte pas le calcul de la pression d'injection.

6. Dispositif d'injection de produit chimique liquide selon l'une quelconque des revendications 1 à 5, dans lequel le dispositif d'injection de produit chimique liquide inclut une console (101) incluant le dispositif d'affichage (103), et une tête d'injection (110) incluant le mécanisme d'entraînement de piston (112, 113) et formée en tant qu'une unité indépendante de la console (101).

7. Dispositif d'injection de produit chimique liquide selon la revendication 6, comprenant en outre un module de communication sans fil (153, 155) pour transmettre et recevoir un signal et des données entre la console (101) et la tête d'injection (110).

8. Dispositif d'injection de produit chimique liquide selon l'une quelconque des revendications 1 à 5, dans lequel la seringue (800) inclut une étiquette RFID (802), des informations relatives au produit chimique liquide introduit et à la seringue (800) étant enregistrées sur l'étiquette RFID (802), et
le système comprend en outre un lecteur (166) configuré pour lire les informations provenant de l'étiquette RFID (802).

9. Dispositif d'injection de produit chimique liquide selon la revendication 8, comprenant en outre un mécanisme de maintien (111) pour maintenir la seringue (800) de manière amovible,
dans lequel le lecteur (166) inclut une antenne (165) située dans une position faisant face à l'étiquette RFID (802) alors que la seringue (800) est maintenue dans le mécanisme de maintien (111).

10. Dispositif d'injection de produit chimique liquide selon la revendication 9, dans lequel l'antenne (165) inclut un circuit imprimé flexible sur lequel est formé un motif constitué d'un conducteur et est pliée dans une forme d'arc concentrique avec la seringue (800).

11. Dispositif d'injection de produit chimique liquide selon la revendication 9 ou 10, dans lequel l'antenne (165) inclut une feuille de ferrite (165a) sur une surface opposée à une surface faisant face à l'étiquette RFID (802).

12. Dispositif d'injection de produit chimique liquide selon l'une quelconque des revendications 1 à 11, comprenant en outre la seringue (800).

13. Dispositif d'injection de produit chimique liquide selon la revendication 12, dans lequel la seringue (800) est une seringue pré-remplie.

14. Dispositif d'injection de produit chimique liquide selon la revendication 12 ou 13, dans lequel la seringue (800) inclut une bride de cylindre (801 ; 501a) formée au niveau d'une extrémité arrière du cylindre (501), et la bride de cylindre (801 ; 501a) inclut une partie encoche (505) sur une partie périphérique externe.

15. Dispositif d'injection de produit chimique liquide selon la revendication 1, comprenant en outre une seringue vide ne contenant pas de produit chimique liquide, dans lequel le circuit de commande du dispositif d'injection (152, 158) est configuré pour permettre le remplissage de la seringue vide avec un produit chimique liquide depuis un conteneur de produit chimique liquide externe en actionnant le mécanisme d'entraînement de piston (112, 113) de sorte que le piston est déplacé vers l'arrière par rapport au cylindre.

16. Dispositif d'injection de produit chimique liquide selon la revendication 15, dans lequel la seringue vide inclut une étiquette RFID, et
le dispositif d'injection de produit chimique liquide comprend en outre un dispositif d'écriture pour écrire des informations concernant le produit chimique liquide introduit sur l'étiquette RFID fournie pour la seringue vide.

EXAMINATION ROOM          OPERATION ROOM

Fig. 1

Fig. 2

Fig. 3

Fig. 4

Fig. 5

Fig. 6

Fig. 7

Fig. 8

Fig. 9

Fig. 10A

300

Fig. 10B

300

Fig. 10C

300

Fig. 10D

300

Fig. 10E

300

Fig. 10F

Fig. 10G

(a)

501b

501

501a

505

502

(b)

501c

501a

501

Fig. 11

Fig. 12

Fig. 13

110

112

Fig. 14

930

920

CHEMICAL LIQUID FILLING
DEVICE

CHEMICAL
LIQUID
CONTAINER

922a — READER ⟷ RFID TAG

922b — WRITER

932

910

DISCARD BOX

READER/WRITER

912

802

800

SYRINGE

RFID TAG

900

WARMER

READER/WRITER

902

100

CHEMICAL LIQUID
INJECTOR

200

IMAGIN
DIAGNOSTIC
APPARATUS

Fig. 15

1
•

2
••

3
• •

4

5

68mL   TOTAL AMOUNT

PRESSURE LIMIT   10kg/cm²

3. 0
mL/sec

100
mL

Fig. 16A

1
•

2
••

3
• •

4

5

68mL   TOTAL AMOUNT

PRESSURE LIMIT   10kg/cm²

1. 0
mL/sec

1
mL

1. 0
mL/sec

1
mL

Fig. 16B

Fig. 16C

Fig. 17

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010204574 A
- WO 2011206399 A

- WO 2011136218 A